# EUROPÄISCHE PATENTSCHRIFT

(11) **EP 1 066 251 B1**
(45) Veröffentlichungstag und Bekanntmachung des Hinweises auf die Patenterteilung: **09.07.2003**
(21) Anmeldenummer: 99913140.2
(22) Anmeldetag: 04.02.1999
(51) Int. Cl.: C07C 277/08, C07C 279/36, C07D 213/61, C07D 277/32, C07D 307/14

(54) **VERFAHREN ZUR HERSTELLUNG VON 1,3-DISUBSTITUIERTEN 2-NITROGUANIDINEN**
METHOD FOR THE PRODUCTION OF 1,3-DISUBSTITUTED 2-NITROGUANIDINES
PROCEDE DE FABRICATION DE 2-NITROGUANIDINES 1,3-DISUBSTITUEES

(30) Priorität: 17.02.1998 DE 19806469
(43) Veröffentlichungstag der Anmeldung: 10.01.2001
(73) Patentinhaber: Bayer CropScience AG, 40789 Monheim (DE)
(72) Erfinder: VAN LAAK, Kai, D-51061 Köln (DE); WOLLWEBER, Detlef, D-42113 Wuppertal (DE)
(86) Internationale Anmeldenummer: EP9900744
(87) Internationale Veröffentlichungsnummer: WO99042437

(56) Entgegenhaltungen:
- EP-A- 0 483 062
- EP-A- 0 869 120
- WO-A-98/56764
- WO-A-99/09009
- DATABASE WPI Section Ch, Week 9206 Derwent Publications Ltd., London, GB; Class C02, AN 92-045988 XP002105582 & JP 03 291267 A (AGRO KANESHO KK) , 20. Dezember 1991 in der Anmeldung erwähnt

## Beschreibung

Die vorliegende Erfindung betrifft ein neuartiges Verfahren zur Herstellung von 1,3-disubstituierten 2-Nitroguanidinen.

Aus EP-A-0 483 062 ist ein Verfahren zur Herstellung von 1,3-disubstituierten 2-Nitroguanidinen bekannt. Sie werden erhalten durch Hydrolyse entsprechender 2-Nitroimino-1,3,5-triazacyclohexanderivate. Die Hydrolyse wird bevorzugt in Gegenwart starker Mineralsäuren oder organischer Säuren durchgeführt.

Nachteilig bei diesem Verfahren sind die langen Reaktionszeiten und das Entstehen von Nebenprodukten, die eine aufwendige Reinigung der gewünschten Endprodukte erforderlich machen.

Darüber hinaus müssen beim Arbeiten in Gegenwart starker Säuren bekanntlich Maßnahmen zum Schutz beispielsweise der Reaktoren gegen Korrosion getroffen werden.

Die JP-03 291 267 betrifft ein ähnliches Verfahren.

Aufgabe der vorliegenden Erfindung war es, ein verbessertes Verfahren zur Herstellung von 1,3-disubstituierten 2-Nitroguanidinen bereitzustellen.

Gegenstand der vorliegenden Erfindung ist ein Verfahren zur Herstellung von Verbindungen der Formel (I) 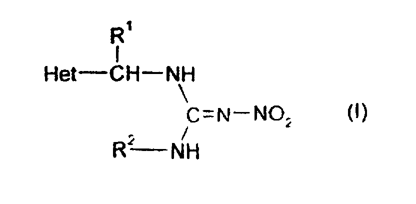 worin
- R¹: für Wasserstoff oder C₁-C₄-Alkyl steht,
- R²: für Wasserstoff, C₁-C₆-Alkyl, C₃-C₆-Cycloalkyl oder einen Rest -CH₂R³ steht,
- R³: für C₂-C₅-Alkenyl, C₂-C₅-Alkinyl, Phenyl, Cyanophenyl, Nitrophenyl, Halogenphenyl mit 1 bis 3 Halogenatomen, durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl, 3-Pyridyl, 5-Thiazolyl, durch ein bis zwei (bevorzugt einen) Substituenten aus der Gruppe C₁-C₃-Alkyl, C₁-C₃-Halogenalkyl mit 1 bis 7 Halogenatomen, Cyclopropyl, Halogencyclopropyl, C₂-C₃-Alkenyl, C₂-C₃-Alkinyl, C₁-C₃-Alkoxy, C₂-C₃-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Halogen, Cyano oder Nitro substituiertes 5-Thiazolyl; oder durch ein bis vier (bevorzugt ein oder zwei) Reste aus der Gruppe C₁-C₃-Halogenalkyl mit 1 bis 7 Halogenatomen, Cyclopropyl, Halogencyclopropyl, C₂-C₃-Alkenyl, C₂-C₃-Alkinyl, C₂-C₃-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Cyano, Nitro, C₁-C₃-Alkyl, C₁-C₃-Alkoxy oder Halogen substituiertes 3-Pyridyl steht,
- Het: für einen unsubstituierten oder substituierten aromatischen oder nichtaromatischen, monocyclischen oder bicyclischen heterocyclischen Rest bevorzugt aus der Reihe 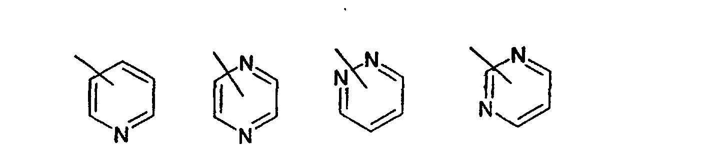 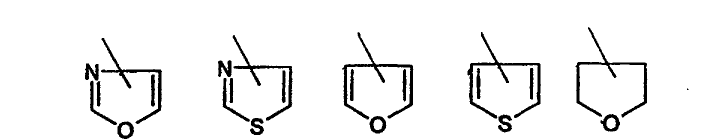 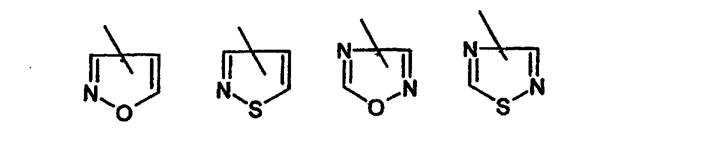 steht, der ein oder zwei Substituenten aus der Gruppe C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₂-C₃-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Cyano, Nitro, C₁-C₃-Alkyl, C₁-C₃-Alkoxy und Halogen enthalten kann,
dadurch gekennzeichnet, daß man eine Verbindung der Formel (II) 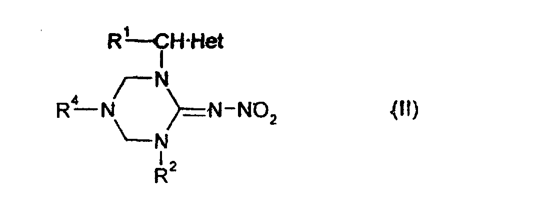 worin
- R¹, R² und Het: die oben angegebenen Bedeutungen haben und
- R⁴: für jeweils unsubsituiertes oder substituiertes C₁-C₁₀-Alkyl, C₃-C₆-Cycloalkyl, Phenyl, Arylalkyl oder Heterocyclylmethyl, wobei Heterocyclyl für einen ungesättigten oder gesättigten 5- oder 6-gliedrigen Heterocyclus mit einem oder mehreren Heteroatomen aus der Reihe Stickstoff, Sauerstoff, Schwefel wie z.B. Furan, Tetrahydrofuran, Thiophen oder Pyridin steht,
gegebenfalls in Gegenwart eines Verdünnungsmittels mit Verbindungen der Formel (III)

R⁵-NH₂ (III)

in welcher
- R⁵: für jeweils gegebenenfalls substituiertes Alkyl, Cycloalkyl, Aryl, Arylalkyl oder Heterocyclylmethyl steht, wobei Heterocyclyl für einen ungesättigten oder gesättigten 5- oder 6-gliedrigen Heterocyclus mit einem oder mehreren Heteroatomen aus der Reihe Stickstoff, Sauerstoff, Schwefel wie z.B. Furan, Tetrahydrofuran, Thiophen oder Pyridin steht,
umsetzt, wobei die Verbindungen der Formel (III) in einem molaren Verhältnis von 0,5:1 bis 10:1 bezogen auf die Ausgangsverbindung der Formel (II) eingesetzt wird.

Die Verbindungen der Formel (I) können auch als Doppelbindungsisomere bezüglich der -N=C(2)-Bindung und in ihren tautomeren Formen (Formeln Ia, Ib) auftreten: 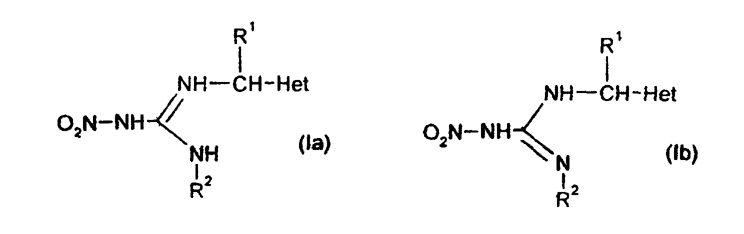 Formel (I) ist demnach so zu verstehen, daß sie auch die entsprechenden Doppelbindungsisomeren und die Formeln (Ia) und (Ib) einschließt.

Überraschenderweise liefert das erfindungsgemäße Verfahren selektiv und in hohen Ausbeuten die Endprodukte der Formel (I) nach kurzer Reaktionszeit unter milden Reaktionsbedingungen in reiner Form.

Verwendet man beispielsweise 1-(2-Chlorthiazol-5-ylmethyl)-2-nitro-imino-5-benzyl-3-methyl-1,3,5-triazacyclohexan als Ausgangsstoff und Benzylamin, so kann der Verlauf des erfindungsgemäßen Verfahrens durch das folgende Formelschema wiedergegeben werden:

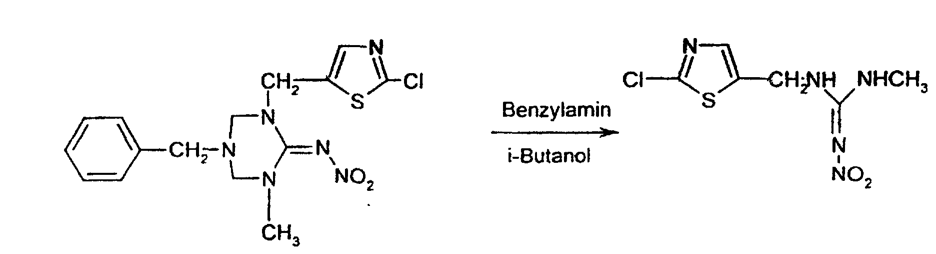

Die als Ausgangsstoffe für das erfindungsgemäße Verfahren benötigten Verbindungen sind durch die Formel (II) allgemein definiert.

Bevorzugte Substituenten bzw. Bereiche der in den oben und nachstehend-erwähnten Formeln aufgeführten Reste werden im folgenden erläutert:
- R¹: steht bevorzugt für Wasserstoff, Methyl, Ethyl, n- oder i-Propyl,
- R²: steht bevorzugt für Wasserstoff, Methyl, Ethyl, n-Propyl, i-Propyl oder n-Butyl, Cyclopropyl, Cyclopentyl, Cyclohexyl oder für einen Rest -CH₂R³,
- R³: steht bevorzugt für C₂-C₅-Alkenyl, C₂-C₅-Alkinyl, Phenyl, Cyanophenyl, Nitrophenyl, Halogenphenyl mit 1 bis 3 Halogenatomen, durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl, 3-Pyridyl, 5-Thiazolyl, durch ein bis zwei (bevorzugt einen) Substituenten aus der Gruppe C₁-C₃-Alkyl, C₁-C₃-Halogenalkyl mit 1 bis 7 Halogenatomen, Cyclopropyl, Halogencyclopropyl, C₂-C₃-Alkenyl, C₂-C₃-Alkinyl, C₁-C₃-Alkoxy, C₂-C₃-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Halogen, Cyan oder Nitro substituiertes 5-Thiazolyl; oder durch ein bis zwei (bevorzugt einen) Reste aus der Gruppe C₁-C₃-Halogenalkyl mit 1 bis 7 Halogenatomen, Cyclopropyl, Halogencyclopropyl, C₂-C₃-Alkenyl, C₂-C₃-Alkinyl, C₂-C₃-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Cyano, Nitro, C₁-C₃-Alkyl, C₁-C₃-Alkoxy oder Halogen substituiertes 3-Pyridyl,
- R⁴: steht bevorzugt für C₁-C₁₀-Alkyl, C₃-C₆-Cycloalkyl, durch 1 bis 6 Reste aus der Gruppe Halogen, Hydroxy, C₁-C₄-Alkoxy, C₁-C₄-Halogenalkoxy mit 1 bis 9 Halogenatomen, Di-( C₁-C₄-Alkyl)-amino oder C₁-C₅-Alkoxycarbonyl substituiertes C₁-C₁₀-Alkyl, durch 1 bis 4 Reste aus der Reihe C₁-C₄-Alkyl oder Halogen substituiertes C₃-C₆-Cycloalkyl, Phenyl, Benzyl, oder durch 1 bis 3 Ringsubstituenten aus der Gruppe Halogen, C₁-C₄-Alkyl, C₁-C₄-Halogenalkyl mit 1 bis 9 Halogenatomen, C₁-C₄-Alkoxy, C₁-C₄-Halogenalkoxy mit 1 bis 9 Halogenatomen, C₁-C₄-Alkylthio, Nitro oder Cyano substituiertes Phenyl oder Benzyl, oder Heterocyclylmethyl, wobei Heterocyclyl für einen ungesättigten oder gesättigten 5- oder 6-gliedrigen Heterocyclus mit einem oder zwei (bevorzugt einem) Heteroatomen aus der Reihe Stickstoff, Sauerstoff, Schwefel wie z.B. Furan, Tetrahydrofuran, Thiophen oder Pyridin steht.
- Het: steht bevorzugt für einen ursubstituierten oder einfach oder zweifach (bevorzugt einfach) substituierten heterocyclischen Rest aus der Reihe 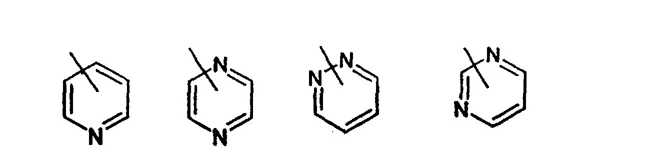 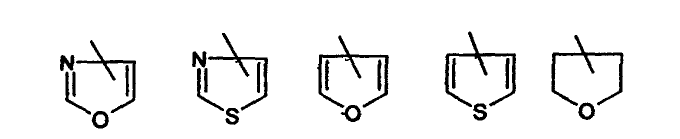 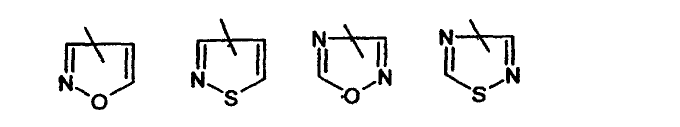 insbesondere für 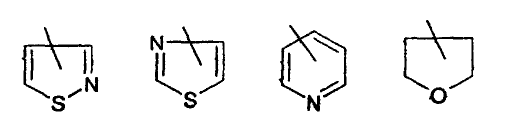 wobei die Substituenten aus der Reihe Fluor, Chlor, Brom, Methyl, Ethyl, Methoxy und Ethoxy ausgewählt sind;
- R⁵: steht bevorzugt für C₁-C₁₀-Alkyl, C₃-C₁₀-Cycloalkyl, Aryl-C₁-C₄-alkyl, insbesondere Phenyl-C₁-C₄-alkyl, jeweils gegebenenfalls einfach oder mehrfach substituiert, wobei als Substituenten NH₂, OH, SH, Halogen, C₁-C₆-Alkoxy, C₁-C₆-Alkylthio, C₁-C₆-Alkyl und Aryl, insbesondere Phenyl in Frage kommen, oder für Heterocyclylmethyl, wobei Heterocyclyl für einen ungesättigten oder gesättigten 5- oder 6-gliedrigen Heterocyclus mit einem oder zwei (bevorzugt einem) Heteroatomen aus der Reihe Stickstoff, Sauerstoff, Schwefel wie z.B. Furan, Tetrahydrofuran, Thiophen oder Pyridin steht,
Halogen(atome) steht bevorzugt für F, Cl, Br, I, insbesondere für F, Cl, Br und hervorgehoben für F, Cl.
- R¹: steht besonders bevorzugt für Wasserstoff, Methyl oder Ethyl,
- R²: steht besonders bevorzugt für Wasserstoff, Methyl, Ethyl, n-Propyl, i-Propyl, n-Butyl, Cyclopropyl, Cyclopentyl, Cyclohexyl oder für einen Rest -CH₂R³,
- R³: steht besonders bevorzugt für C₂-C₃-Alkenyl, C₂-C₃-Alkinyl, Phenyl, Cyanophenyl, Nitrophenyl, Halogenphenyl mit 1 bis 3 Halogenatomen, durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl, 3-Pyridyl, 5-Thiazolyl, jeweils durch ein oder zwei (bevorzugt einen) Substituenten aus der Gruppe C₁-C₃-Alkyl, C₁-C₃-Halogenalkyl mit 1 bis 7 Halogenatomen, C₁-C₃-Alkoxy, C₁-C₃-Halogenalkoxy mit 1 bis 7 Halogenatomen, C₁-C₃-Alkylthio, C₁-C₃-Halogenalkylthio mit 1 bis 7 Halogenatomen, Halogen, Cyano oder Nitro substituiertes 5-Thiazolyl oder 3-Pyridyl,
- R⁴: steht besonders bevorzugt für C₁-C₁₀-Alkyl, C₃-C₆-Cycloalkyl, durch 1 bis 6 Reste aus der Gruppe Halogen, C₁-C₄-Alkoxy und C₁-C₄-Haloalkoxy mit 1 bis 9 Halogenatomen substituiertes C₁-C₈-Alkyl, durch 1 oder 2 Reste aus der Reihe Methyl, Ethyl, Fluor und Chlor substituiertes C₃-C₆-Cycloalkyl, Phenyl, Benzyl, oder jeweils durch 1 bis 3 Ringsubstituenten aus der Gruppe Halogen. C₁-C₄-Alkyl, C₁-C₄-Halogenalkyl mit 1 bis 9 Halogenatomen, C₁-C₄-Alkoxy, C₁-C₄-Halogenalkoxy mit 1 bis 9 Halogenatomen, C₁-C₄-Alkylthio, Nitro oder Cyano substituiertes Phenyl oder Benzyl, oder Heterocyclylmethyl, wobei Heterocyclyl für einen ungesättigten oder gesättigten 5 - oder 6-gliedrigen Heterocyclus mit einem Heteroatom aus der Reihe Stickstoff, Sauerstoff und Schwefel wie z.B. Furan, Tetrahydrofuran, Thiophen oder Pyridin steht.
- Het: steht besonders bevorzugt für jeweils unsubstituiertes oder einfach oder zweifach (insbesondere einfach) substituiertes Thiazolyl, Pyridyl oder Tetrahydrofuranyl, wobei die Substituenten aus der Reihe Fluor, Chlor, Methyl und Methoxy ausgewählt sind,
- R⁵: steht besonders bevorzugt für C₁-C₄-Alkyl, C₃-C₆-Cycloalkyl, Benzyl oder Phenylethyl, jeweils gegebenenfalls einfach bis fünffach substituiert, wobei als Substituenten, NH₂, OH, Cl, Br, F, C₁-C₄-Alkoxy, C₁-C₄-Alkylthio, C₁-C₄-Alkyl und Aryl, insbesondere Phenyl in Frage kommen, oder für Heterocyclylmethyl, wobei Heterocyclyl für einen ungesättigten oder gesättigten 5- oder 6-gliedrigen Heterocyclus mit einem Heteroatom aus der Reihe Stickstoff, Sauerstoff, Schwefel wie z.B. Furan, Tetrahydrofuran, Thiophen oder Pyridin steht,
Halogen(atome) steht besonders bevorzugt für F, Cl, Br, I, insbesondere F, Cl, Br, besonders hervorgehoben für F. Cl.
- R¹: steht ganz besonders bevorzugt für Wasserstoff, Methyl oder Ethyl, hervorgehoben für Wasserstoff,
- R²: steht ganz besonders bevorzugt für Wasserstoff, Methyl, Ethyl, n-Propyl, Cyclopropyl, Cyclopentyl, Allyl, Propargyl oder p-Chlorbenzyl,
- R⁴: steht ganz besonders bevorzugt für Methyl, Ethyl, n-Propyl, Cyclopropyl, Cyclopentyl, Cyclohexyl, Phenyl, Benzyl oder Furfurylmethyl,
- Het: steht ganz besonders bevorzugt für einen der Reste 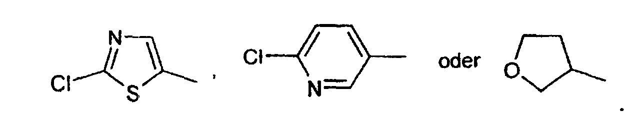
- R⁵: steht ganz besonders bevorzugt für den jeweiligen Rest R⁴ der umzusetzenden Verbindung der Formel (II) oder die Reste Benzyl, NH₂-CH₂-CH₂-, HO-CH₂-CH₂-, n-Hexyl oder Cyclohexyl.

Besonders hervorgehoben seien als Ausgangsstoffe für das erfindungsgemäße Verfahren Verbindungen der Formel (IIa) 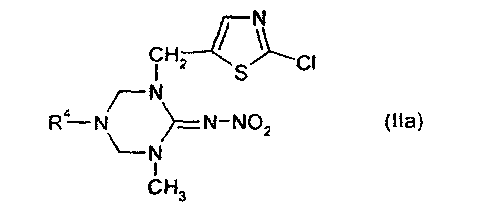 worin
- R⁴: für Methyl, Ethyl, Cyclopropyl, Cyclopentyl, Benzyl oder Furfurylmethyl steht, wobei unter diesen wiederum Methyl, Benzyl und Furfurylmethyl bevorzugt sind.

Besonders hervorgehoben seien als Ausgangsstoffe für das erfindungsgemäße Verfahren Verbindungen der Formel (IIb) und (llc) 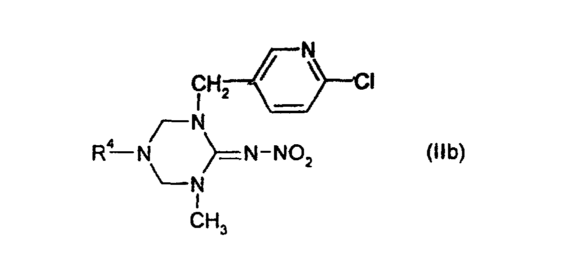 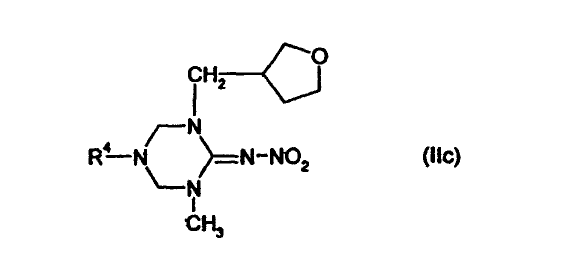 worin
- R⁴: die oben für die Verbindungen der Formel (IIa) angegebenen Bedeutungen hat.

Als Endprodukte des erfindungsgemäßen Verfahrens erhält man bei Verwendung der Verbindung der Formel (IIa) die folgende Verbindung 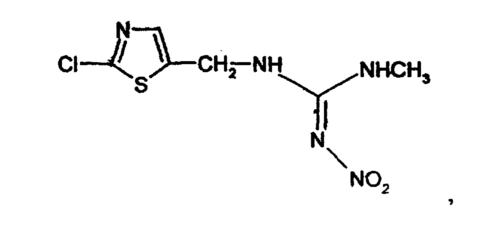 bei Verwendung der Verbindung der Formel (IIb) die folgende Verbindung 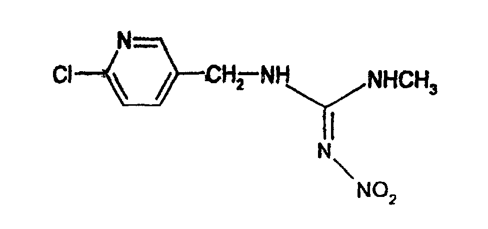 und bei Verwendung der Verbindung der Formel (IIc) die folgende Verbindung

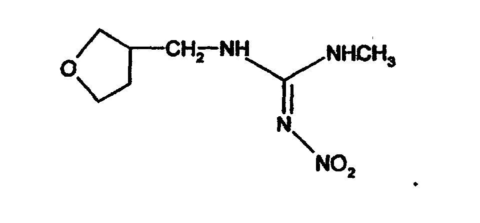

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Unter der Bezeichnung Alkyl sind dabei auch die verzweigten Isornere, z.B. t-Butyl für C₄-Alkyl, zu verstehen.

Bevorzugt werden diejenigen Verbindungen der Formel (II) in das erfindungsgemäße Verfahren eingesetzt, in welchen eine Kombination der vorstehend als bevorzugt aufgeführten Bedeutungen vorliegt.

Besonders bevorzugt werden diejenigen Verbindungen der Formel (II) in das erfindungsgemäße Verfahren eingesetzt, in weichen eine Kombination der vorstehend als besonders bevorzugt aufgeführten Bedeutungen vorliegt.

Ganz besonders bevorzugt werden diejenigen Verbindungen der Formel (II) in das erfindungsgemäße Verfahren eingesetzt, in welchen eine Kombination der vorstehend als ganz besonders bevorzugt aufgeführten Bedeutungen vorliegt.

Die Ausgangsstoffe der Formel (II) sind bekannt oder können nach bekannten Verfahren hergestellt werden (vgl. EP-A-0 483 062, JP-03 291 267, EP-A-0 483 055, EP-A-0 428 941, EP-A-0 386 565).

Man erhält die Verbindungen der Formel (II) beispielsweise, wenn man
a) eine Verbindung der Formel (IV) 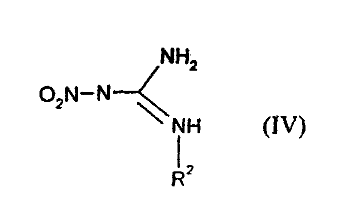 mit Formaldehyd und einer Verbindung der Formel (V)

   H₂N-R⁴ (V)

   umsetzt; und
b) die erhaltene Verbindung der Formel (VI) 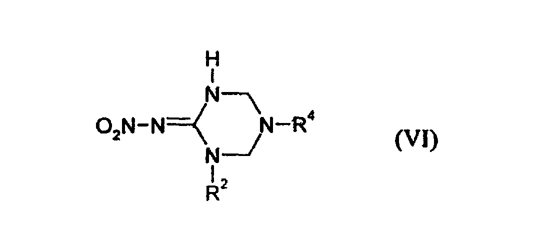 mit einer Verbindung der Formel (VH) 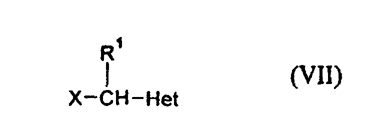 umsetzt, wobei in den Formeln (II) bis (VII) die Reste R¹, R², R⁴ und Het die oben angegebenen Bedeutungen haben und X für eine Abgangsgruppe steht.

Als Abgangsgruppe X können z.B. in Betracht kommen: Halogen, vorzugsweise Chlor, Brom oder Jod, oder Sulfonsäurereste, wie Alkylsulfonsäurereste, Mesylat oder Tosylat.

Die Durchführung der Stufe a) des obigen Verfahrens zur Herstellung der Verbindungen der Formel (II) erfolgt mit Vorteil unter normalem Druck, gegebenenfalls auch unter erhöhtem Druck und gegebenenfalls in einem geeigneten Lösungsmittel und bei Temperaturen zwischen 0°C und +140°C, vorzugsweise zwischen +20°C und +120°C. Als Lösungsmittel eignen sich in besonderer Weise Alkohole, wie Methanol, Ethanol und Propanol, sowie Wasser. Weitere geeignete Lösungsmittel sind z.B. aromatische Kohlenwasserstoffe, wie Benzol, Toluol oder Xylol; Ether wie Tetrahydrofuran, Dioxan oder Diethylether; halogenierte Kohlenwasserstoffe, wie Methylenchlorid, Chloroform, Tetrachlorkohlenstoff oder Chlorbenzol oder andere Lösungsmittel, die die Reaktion nicht beeinträchtigen. Die Lösungsmittel können auch als Gemische verwendet werden. Die Verfahrensstufe b) des obigen Verfahrens zur Herstellung von Verbindungen der Formel (II) kann vorzugsweise unter normalem oder leicht erhöhtem Druck und in Gegenwart von vorzugsweise aprotischen Lösungs- oder Verdünnungsmitteln durchgeführt werden. Als Lösungs- oder Verdünnungsmittel eignen sich z.B. Ether oder etherartige Verbindungen, wie Diethylether, Dipropylether, Dibutylether, Dioxan, Dimethoxyether oder Tetrahydrofuran; aliphatische, aromatische oder halogenierte Kohlenwasserstoffe, insbesondere Benzol, Toluol, Xylol, Chloroform, Methylenchlorid, Tetrachlorkohlenstoff oder Chlorbenzol; Nitrile, wie Acetonitril oder Propionitril; Dimethylsulfoxid oder Dimethylfonnamid. Die Lösungsmittel können auch als Gemische verwendet werden. Diese Verfahrensstufe wird im allgemeinen bei einer Temperatur von -20°C bis +140°C, vorzugsweise zwischen 0°C und +120°C, vorzugsweise in Gegenwart einer Base, durchgeführt. Als Basen eignen sich z.B. Carbonate, wie Natrium- oder Kaliumcarbonat. Auch Hydride, wie Natriumhydrid, Kaliumhydrid oder Calciumhydrid, können als Basen eingesetzt werden. Gegebenenfalls kann die Umsetzung auch in Gegenwart eines Katalysators, z.B. Cäsiumchlorid, durchgeführt werden.

Die Ausgangsprodukte der obigen Formeln (V) und (VII) sind bekannt und im Handel erhältlich oder können leicht in Analogie zu bekannten Verfahren hergestellt werden. Die 2-Nitroguanidin-Ausgangsprodukte der Formel (IV) sind ebenfalls bekannt; sie lassen sich vorteilhaft aus S-Methyl-N-nitroisothioharnstoff durch Umsetzung mit einem entsprechenden primären Amin herstellen (vgl. US-PS 4 804 780 und 4 221 802). N-Methyl-N'-nitroguanidin ist auch durch Umsetzung von Nitroguanidin mit Methylamin erhältlich (vgl. EP-0 798 293). S-Methyl-N-nitroisothioharnstoff erhält man in guter Ausbeute durch Nitrierung von S-Methylisothioharnstoff (vgl. J. Am. Soc. 76, 1877 (1954)).

Die Verbindungen der Formel (III) sind bekannt und im Handel erhältlich oder können leicht nach bekannten Verfahren hergestellt werden. Vorzugsweise werden solche Verbindungen der Formel (III) eingesetzt, in denen R⁵ dem Rest R⁴ der umzusetzenden Verbindungen der Formel (II) entspricht.

Das erfindungsgemäße Verfahren wird gegebenenfalls in Gegenwart eines Verdünnungsmittels duschgeführt.

Als Verdünnungsmittel eignen sich insbesondere organische Lösungsmittel, besonders polare protische Lösungsmittel, beispielsweise Alkohole wie Methanol, Ethanol, n-Propanol, i-Propanol, n-Butanol oder i-Butanol und polare aprotische Lösungsmittel, beispielsweise Amide wie Dimethylformamid, Dimethylacetamid oder N-Methylpyrrolidon oder Sulfoxide wie Dimethylsulfoxid.

Es ist auch möglich, Gemische der genannten Verdünnungsmittel einzusetzen. Besonders als Verdünnungsmittel geeignet sind Alkohole, insbesondere Methanol, Ethanol, Propanol, i-Propanol, n-Butanol, i-Butanol, sec.-Butanol.

Es ist auch möglich in einem zweiphasigen Gemisch aus Wasser und einem der genannten Verdünnungsmittel zu arbeiten.

Es kann von Vorteil sein, der Reaktionsmischung ein weiteres Lösungsmittel zuzusetzen. Als solches kommen Ether in Frage, beispielsweise Dibutylether, Tetrahydrofuran, Dioxan, Glykoldimethylether oder Diglykoldimethylether, weiterhin Kohlenwasserstoffe, wie Benzol, Toluol oder Xylol, halogenierte Kohlenwasserstoffe, wie Methylenchlorid, Chloroform, Tetrachlorkohlenstoff, Chlorbenzol oder o-Dichlorbenzol, Nitrile wie Acetonitril, Carbonsäureester wie Ethylacetat oder auch Ketone wie Aceton oder Methylisopropylketon.

Es ist auch möglich, Gemische der genannten Lösungsmittel einzusetzen.

Das erfindungsgemäße Verfahren wird bei Temperaturen zwischen 0°C und 200°C, vorzugsweise zwischen 40°C und 150°C, durchgeführt.

Vorzugsweise wird unter Normaldruck gearbeitet, im Falle niedrigsiedender oder gasförmiger Verbindungen der Formel (III) sowie im Falle niedrigsiedender Verdünnungmittel kann gegebenenfalls auch unter erhöhtem Druck gearbeitet werden.

Die Verbindungen der Formel (III) werden in einem molaren Verhältnis von 0,5:1 bis 10:1, vorzugsweise 1:1 bis 5:1, bezogen auf die Ausgangsverbindung der Formel (II), eingesetzt.

Im allgemeinen wird die Umsetzung so durchgeführt, daß man den Ausgangsstoff der Formel (II) und die Verbindung der Formel (III) gegebenenfalls in einem Verdünnungsmittel und gegebenenfalls im Lösungsmittel auf die gewünschte Temperatur erhitzt. Es ist auch möglich, die Verbindung der Formel (III) im Verlauf der Reaktion sukzessive einzudosieren.

Zur Aufarbeitung wird nach dem Abkühlen gegebenenfalls mit Wasser versetzt und das Endprodukt gegebenenfalls nach Einengen des Gemischs beispielsweise durch Filtration oder Extraktion isoliert.

Bevorzugt wird die Reaktion in einem Verdünnungsmittel durchgeführt, aus welchem beim Abkühlen der Reaktionsmischung das Endprodukt direkt auskristallisiert und in einfacher Weise beispielsweise durch Filtration isoliert werden kann. Als Verdünnungsmittel kommen hierfür Alkohole in Frage, insbesondere Methanol, Ethanol, Propanol, i-Propanol, Isobutanol, n-Butanol, sec-Butanol.

Es ist auch möglich, eine wasserfreie Aufarbeitung des Reaktionsgemisches durchzuführen, indem man nach beendeter Reaktion gegebenenfalls das Verdünnungsmittel und gegebenenfalls Lösungsmittel abdestilliert und den verbleibenden Rückstand mit einem geeigneten Extraktionsmittel extrahiert. Als Extraktionsmittel kommen grundsätzlich alle gegenüber den Endprodukten inerten Lösungsmittel in Frage, in denen die Endprodukte ausreichend löslich sind.

Hierzu gehören beispielsweise aliphatische Kohlenwasserstoffe wie n-Pentan, n-Hexan, Cyclohexan, halogenierte aliphatische Kohlenwasserstoffe wie Methylenchlorid oder Chloroform, aromatische Kohlenwasserstoffe wie Benzol, Toluol oder Xylol, halogenierte aromatische Kohlenwasserstoffe wie Chlorbenzol oder o-Dichlorbenzol oder auch Ether wie beispielsweise Methyl-tert.-butylether.

Die Endprodukte kristallisieren, gegebenenfalls nach Einengen des Extraktionsmittels, aus und können durch Filtration isoliert werden, oder das Extraktionsmittel wird vollständig oder nahezu vollständig entfernt und der Rückstand, falls nötig, beispielsweise durch Umkristallisation gereinigt.

Die erfindungsgemäß hergestellten Verbindungen der Formel (I) sind wertvolle Wirkstoffe in der Schädlingsbekämpfung. Insbesondere sind die Verbindungen der Formel (I) geeignet zur Bekämpfung von Insekten und Spinnentieren, die in Nutzund Zierpflanzen in der Landwirtschaft, insbesondere in Baumwoll-, Gemüse- und Obstpflanzungen, im Forst, im Vorrats- und Materialschutz sowie im Hygienesektor insbesondere an Haus- und Nutztieren vorkommen (siehe z.B. EP-A-0 376 279, EP-A-0 375 907, EP-A-0 383 091).

### Beispiele

### Beispiel 1

### Herstellung von 1-(2-Chlorthiazol-5-ylmethyl)-2-nitro-3-methylguanidin

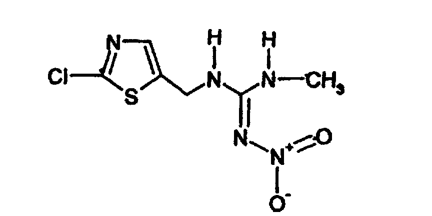

a) 39 g 1-(2-Chlorthiazol-5-ylmethyl)-2-nitro-imino-5-benzyl-3-methyl-1,3,5-triazacyclohexan 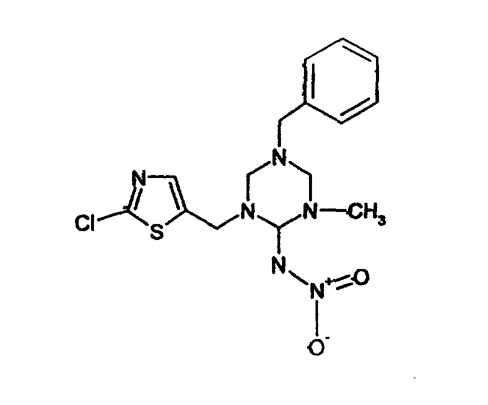 und 16,5 g Benzylamin werden in 200 ml Isobutanol 3 Stunden lang auf 95 bis 100°C erhitzt. Es wird auf 20°C abgekühlt, eine Stunde lang bei 20°C nachgerührt und das Produkt durch Filtration isoliert. Das Kristallisat wird mit Wasser gewaschen und anschließend getrocknet.
   Ausbeute 22 g, HPLC: 95 %.
   Das Produkt ist nach seinen chromatographischen und spektroskopischen Daten identisch mit einer auf anderem Weg erhaltenen, authentischen Probe 1-(2-Chlorthiazol-5-ylmethyl)-2-nitro-3-methylguanidin.
b) 39 g 1-(2-Chlorthiazol-5-ylmethyl)-2-nitro-imino-5-benzyl-3-methyl-1,3,5-triazacyclohexan und 3,6 g Ethylendiamin werden in 200 ml Isobutanol 2 Stunden lang auf 95-100°C erhitzt. Es wird auf 20°C abgekühlt, eine Stunde lang bei 20°C nachgerührt und das Produkt durch Filtration isoliert. Das Kristallisat wird mit Wasser gewaschen und anschließend getrocknet. Ausbeute 21,7 g.
   Das Produkt ist nach seinen chromatographischen Daten identisch mit einer auf anderem Weg erhaltenen, authentischen Probe 1-(2-Chlorthiazol-5-ylmethyl)-2-nitro-3-methylguanidin.
c) 4 g 1-(2-Chlorthiazol-5-ylmethyl)-2-nitro-imino-5-benzyl-3-methyl-1,3,5-triazacyclohexan und 2 g Aminoethanol werden in 20 ml Isobutanol 2 Stunden lang auf 110°C erhitzt.
   Dünnschichtchromatographische Reaktionskontrolle zeigt vollständigen Umsatz zum 1-(2-Chlorthiazol-5-ylmethyl)-2-nitro-3-methylguanidin.
d) 38,4 g 1-(2-Chlorthiazol-5-ylmethyl)-2-nitro-imino-5-benzyl-3-methyl-1,3,5-triazacyclohexan und 12,1 g n-Hexylamin werden in 200 ml n-Butanol 2 Stunden lang auf 95-100°C erhitzt.
   Dünnschichtchromatographische Reaktionskontrolle zeigt vollständigen Umsatz zum 1-(2-Chlorthiazol-5-ylmethyl)-2-nitro-3-methylguanidin.
e) 39,2 g 1-(2-Chlorthiazol-5-ylmethyl)-2-nitro-imino-5-benzyl-3-methyl-1,3,5-triazacyclohexan und 11,9 g Cyclohexylamin werden in 200 ml Isobutanol 4 Stunden lang auf 95-100°C erhitzt.
   Dünnschichtchromatographische Reaktionskontrolle zeigt vollständigen Umsatz zum 1-(2-Chlorthiazol-5-ylmethyl)-2-nitro-3-methylguanidin.
f) 76 g 1-(2-Chlorthiazol-5-ylmethyl)-2-nitro-imino-5-benzyl-3-methyl-1,3,5-triazacyclohexan werden in 150 ml Methanol vorgelegt. Es wird auf 65°C erhitzt und innerhalb von einer Stunde 32 g Benzylamin zum Ansatz dosiert. Es wird 4 Stunden lang bei 65°C nachgerührt. Es wird auf 0°C abgekühlt, eine Stunde lang bei 0°C nachgerührt und das Produkt durch Filtration isoliert. Das Kristallisat wird mit kaltem Methanol gewaschen und anschließend getrocknet.
   Ausbeute 40 g, HPLC: 99 %.
   Das Produkt ist nach seinen chromatographischen und spektroskopischen Daten identisch mit einer auf anderem Weg erhaltenen, authentischen Probe 1-(2-Chlorthiazol-5-ylmethyl)-2-nitro-3-methylguanidin.

Analog können auch die in der folgenden Tabelle aufgeführten Verbindungen der Formel (I) erhalten werden:

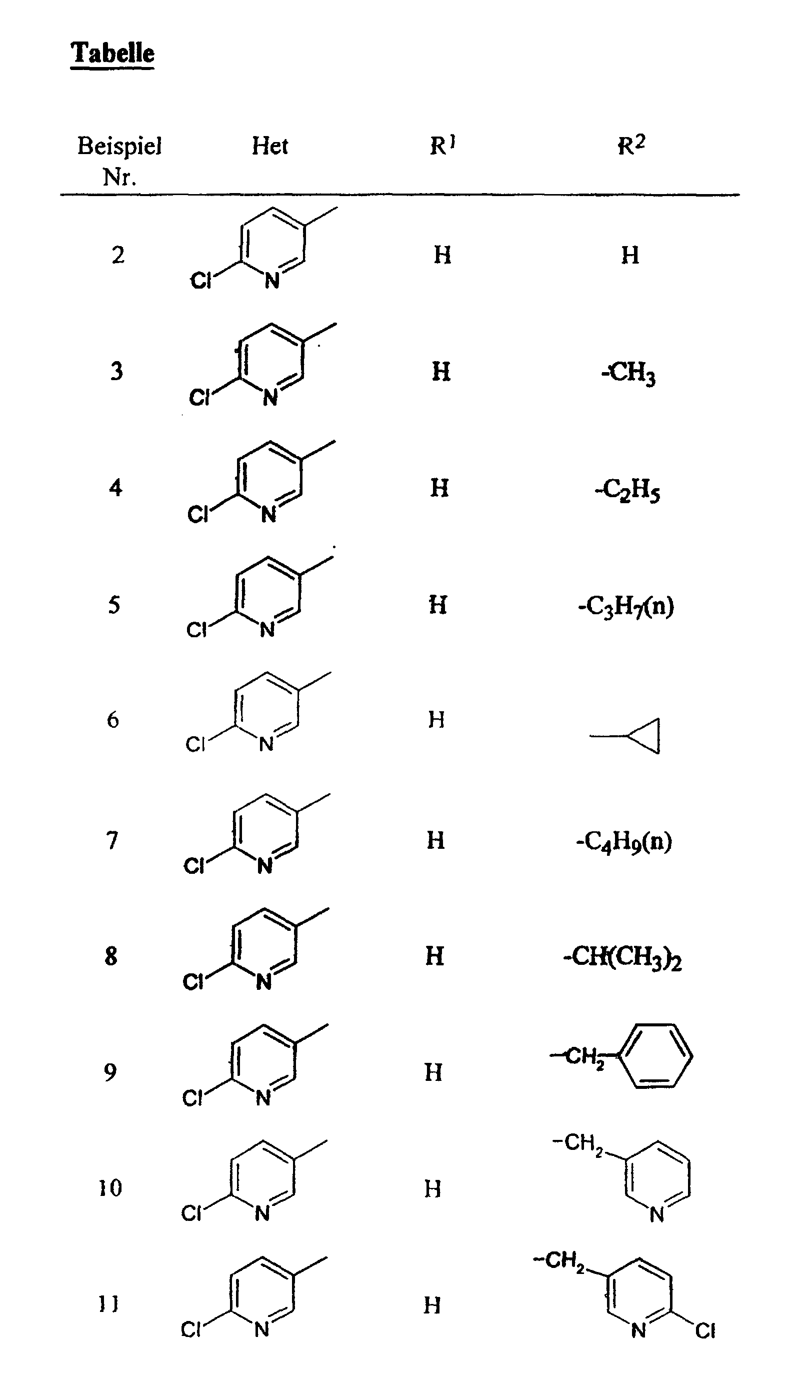

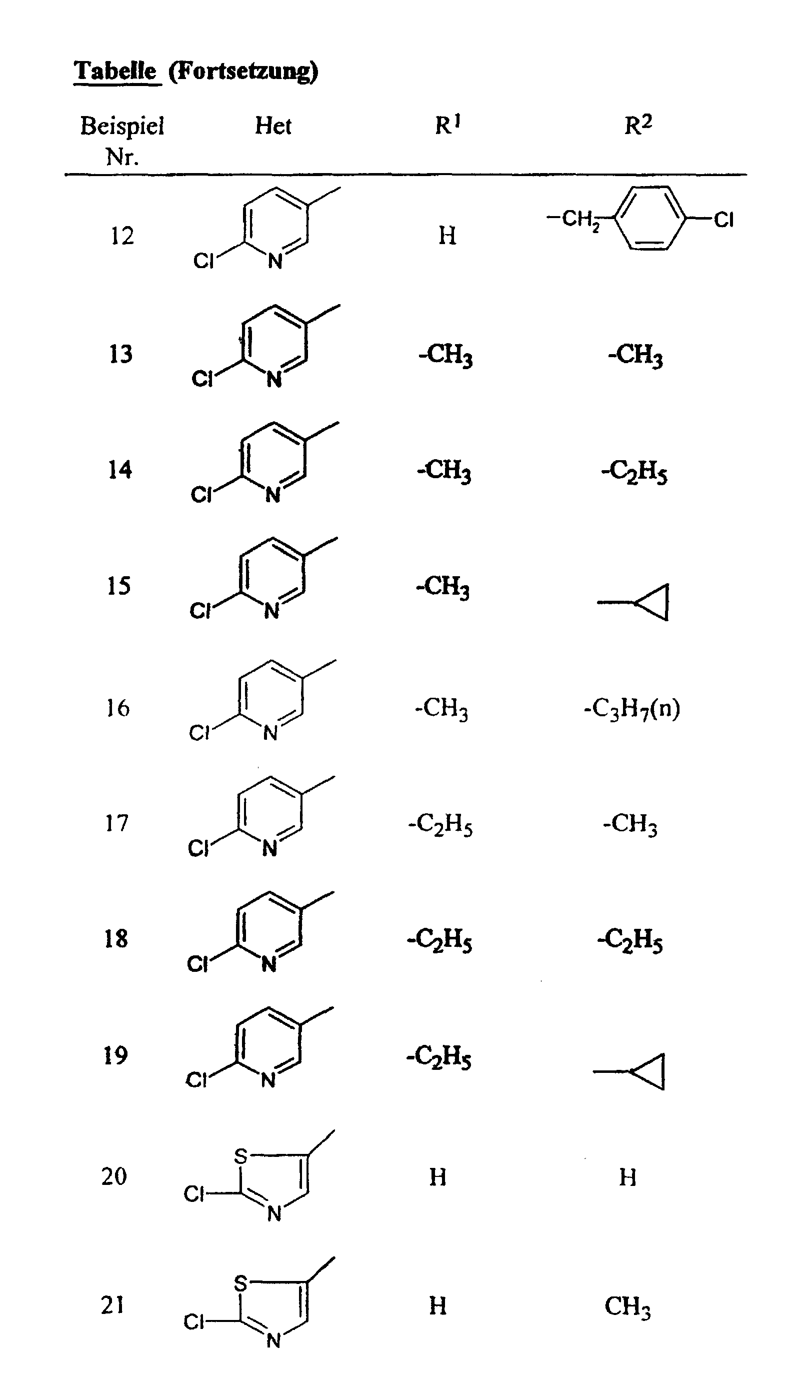

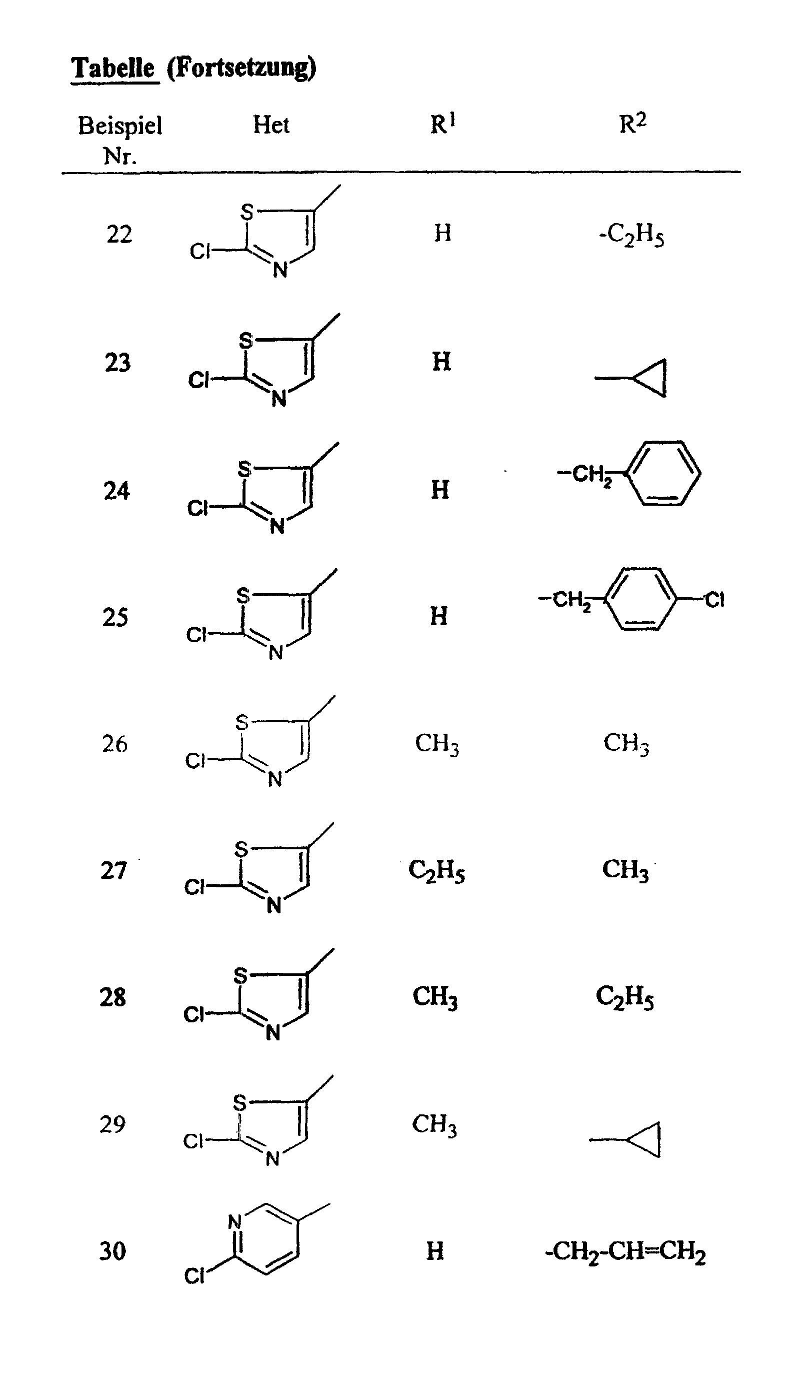

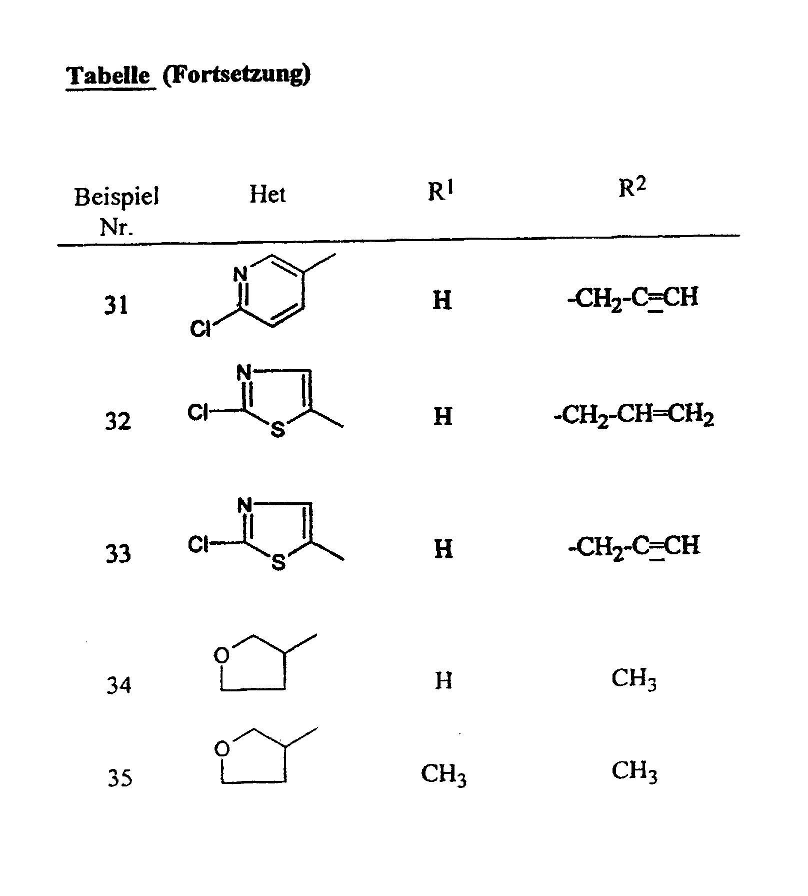

## Patentansprüche

1. Verfahren zur Herstellung von Verbindungen der Formel (I) 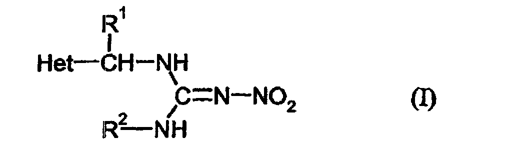 worin
R¹ für Wasserstoff oder C₁-C₄-Alkyl steht,
R² für Wasserstoff, C₁-C₆-Alkyl, C₃-C₆-Cycloalkyl oder einen Rest -CH₂R³ steht,
R³ für C₂-C₅-Alkenyl, C₂-C₅-Alkinyl, Phenyl, Cyanophenyl, Nitrophenyl, Halogenphenyl mit 1 bis 3 Halogenatomen, durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl, 3-Pyridyl, 5-Thiazolyl, durch ein bis zwei (bevorzugt einen) Substituenten aus der Gruppe C₁-C₃-Alkyl, C₁-C₃-Halogenalkyl mit 1 bis 7 Halogenatomen, Cyclopropyl, Halogencyclopropyl, C₂-C₃-Alkenyl, C₂-C₃-Alkinyl, C₁-C₃-Alkoxy, C₂-C₃-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Halogen, Cyano oder Nitro substituiertes 5-Thiazolyl; oder durch ein bis vier (bevorzugt ein oder zwei) Reste aus der Gruppe C₁-C₃-Halogenalkyl mit 1 bis 7 Halogenatomen, Cyclopropyl, Halogencyclopropyl, C₂-C₃-Alkenyl, C₂-C₃-Alkinyl, C₂-C₃-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Cyano, Nitro, C₁-C₃-Alkyl, C₁-C₃-Alkoxy oder Halogen substituiertes 3-Pyridyl steht,
Het für einen ursubstituierten oder substituierten aromatischen oder nicht aromatischen, monocyclischen oder bicyclischen heterocyclischen Rest bevorzugt aus der Reihe 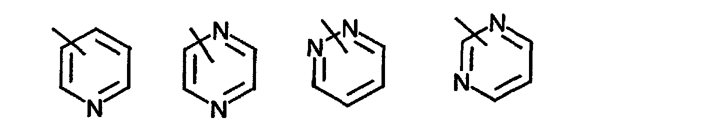 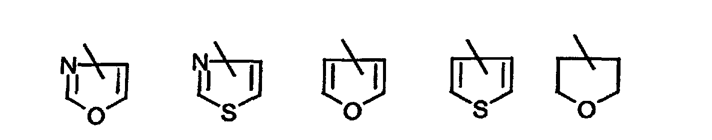 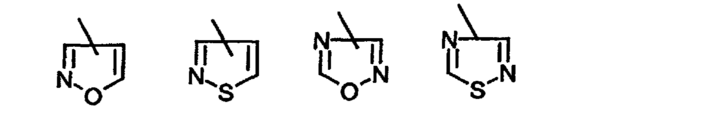 steht, der ein bis zwei Substituenten aus der Gruppe C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C2-C3-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Cyano, Nitro, C₁-C₃-Alkyl, C₁-C₃-Alkoxy und Halogen enthalten kann,
**dadurch gekennzeichnet, dass** man eine Verbindung der Formel (II) 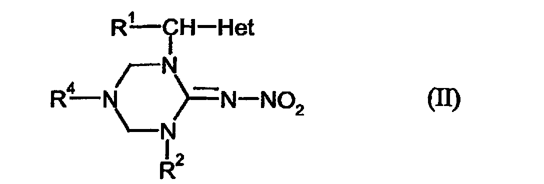 worin
R¹, R² und Het die oben angegebenen Bedeutungen haben und
R⁴ für jeweils unsubsituiertes oder substituiertes C₁-C₁₀-Alkyl, C₃-C₆-Cycloalkyl, Phenyl, Arylalkyl oder Heterocyclylmethyl, wobei Heterocyclyl für cincn ungesättigten oder gesättigten 5- oder 6-gliedrigen Heterocyclus mit einem oder mehreren Heteroatomen aus der Reihe Stickstoff, Sauerstoff, Schwefel wie z.B. Furan, Tetrahydrofuran, Thiophen oder Pyridin steht,
gegebenenfalls in Gegenwart eines Verdünnungsmittels mit Verbindungen der Formel (III), 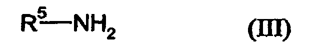 in welcher
R⁵ für gegebenenfalls substituiertes Alkyl, Cycloalkyl, Aryl, Arylalkyl oder Heterocyclylmethyl, wobei Heterocyclyl für einen ungesättigten oder gesättigten 5- oder 6-gliedrigen Heterocyclus mit einem oder mehreren Heteroatomen aus der Reihe Stickstoff, Sauerstoff, Schwefel wie z.B. Furan, Tetrahydrofuran, Thiophen oder Pyridin steht,
umsetzt, wobei die Verbindungen der Formel (III) in einem molaren Verhältnis von 0,5:1 bis 10:1 bezogen auf die Ausgangsverbindung der Formel (II) eingesetzt wird.

2. Verfahren gemäß Anspruch 1, wobei in den Formeln (I), (II) und (III) gemäß Anspruch 1
R¹ für Wasserstoff, Methyl, Ethyl, n- oder i-Propyl steht,
R² für Wasserstoff, Methyl, Ethyl, n-Propyl, i-Propyl oder n-Butyl, Cyclopropyl, Cyclopentyl, Cyclohexyl oder für einen Rest -CH₂R³ steht,
R³ für C₂-C₅-Alkenyl, C₂-C₅-Alkinyl, Phenyl, Cyanophenyl, Nitrophenyl, Halogenphenyl mit 1 bis 3 Halogenatomen, durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl, 3-Pyridyl, 5-Thiazolyl, durch ein bis zwei (bevorzugt einen) Substituenten aus der Gruppe C₁-C₃-Alkyl, C₁-C₃-Halogenalkyl mit 1 bis 7 Halogenatomen, Cyclopropyl, Halogencyclopropyl, C₂-C₃-Alkenyl, C₂-C₃-Alkinyl, C₁-C₃-Alkoxy, C₂-C₃-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Halogen, Cyano oder Nitro substituiertes 5-Thiazolyl steht; oder flir durch ein bis zwei (bevorzugt einen) Reste aus der Gruppe C₁-C₃-Halogenalkyl mit 1 bis 7 Halogenatomen, Cyclopropyl, Halogencyclopropyl, C₂-C₃-Alkenyl, C₂-C₃-Alkinyl, C₂-C₃-Halogenalkenyl mit 1 bis 4 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Cyano, Nitro, C₁-C₃-Alkyl, C₁-C₃-Alkoxy oder Halogen substituiertes 3-Pyridyl steht,
R⁴ für C₁-C₁₀-Alkyl, C₃-C₆-Cycloalkyl, durch 1 bis 6 Reste aus der Gruppe Halogen, Hydroxy, C₁-C₄-Alkoxy, C₁-C₄-Halogenalkoxy mit 1 bis 9 Halogenatomen, Di-(C₁-C₄-Alkyl)-amino oder C₁-C₅-Alkoxycarbonyl substituiertes C₁-C₁₀-Alkyl, durch 1 bis 4 Reste aus der Reihe C₁-C₄-Alkyl oder Halogen substituiertes C₃-C₆-Cycloalkyl, Phenyl, Benzyl, oder durch 1 bis 3 Ringsubstituenten aus der Gruppe Halogen, C₁-C₄-Alkyl, C₁-C₄-Halogenalkyl mit 1 bis 9 Halogenatomen, C₁-C₄-Alkoxy, C₁-C₄-Halogenalkoxy mit 1 bis 9 Halogenatomen, C₁-C₄-Alkylthio, Nitro oder Cyano substituiertes Phenyl, Benzyl oder Heterocyclylmethyl steht, wobei Heterocyclyl für einen ungesättigten oder gesättigten 5- oder 6-gliedrigen Heterocyclus mit einem oder zwei (bevorzugt einem) Heteroatomen aus der Reihe Stickstoff, Sauerstoff, Schwefel wie z.B. Furan, Tetrahydrofuran, Thiophen oder Pyridin steht,
Het für einen unsubstituierten oder einfach oder zweifach (bevorzugt einfach) substituierten heterocyclischen Rest aus der Reihe 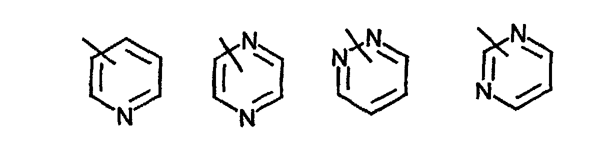 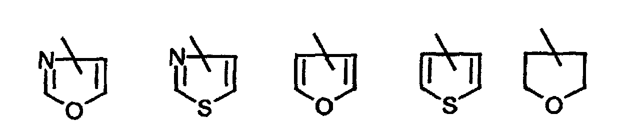 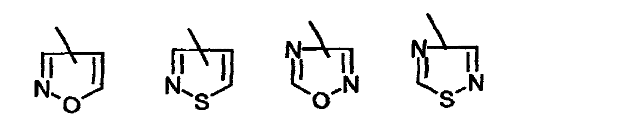 insbesondere für 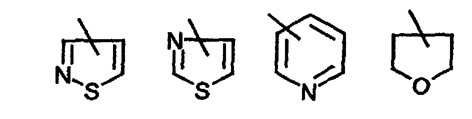 steht, wobei die Substituenten aus der Reihe Fluor, Chlor, Brom, Methyl, Ethyl, Methoxy und Ethoxy ausgewählt sind,
R⁵ für C₁-C₁₀-Alkyl, C₃-C₁₀-Cycloalkyl, Aryl-C₁-C₄-alkyl, insbesondere Phenyl-C₁-C₄-alkyl steht, jeweils gegebenenfalls einfach oder mehrfach substituiert, wobei als Substituenten NH₂, OH, SH, Halogen, C₁-C₆-Alkoxy, C₁-C₆-Alkylthio, C₁-C₆-Alkyl und Aryl, insbesondere Phenyl in Frage kommen, oder für Heterocyclylmethyl steht, wobei Heterocyclyl für einen ungesättigten oder gesättigten 5- oder 6-gliedrigen Heterocyclus mit einem oder zwei (bevorzugt einem) Heteroatomen aus der Reihe Stickstoff, Sauerstoff, Schwefel wie z.B. Furan, Tetrahydrofuran, Thiophen oder Pyridin steht

3. Verfahren gemäß Anspruch 1, wobei in den Formeln (I), (II) und (III) gemäß Anspruch 1
R¹ für Wasserstoff, Methyl oder Ethyl steht,
R² für Wasserstoff, Methyl, Ethyl, n-Propyl, i-Propyl, n-Butyl, Cyclopropyl, Cyclopentyl, Cyclohexyl oder für einen Rest -CH₂R³ steht,
R³ für C₂-C₃-Alkenyl, C₂-C₃-Alkinyl, Phenyl, Cyanophenyl, Nitrophenyl, Halogenphenyl mit 1 bis 3 Halogenatomen, durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl, 3-Pyridyl, 5-Thiazolyl, durch ein bis zwei (bevorzugt einen) Substituenten aus der Gruppe C₁-C₃-Alkyl, C₁-C₃-Halogenalkyl mit 1 bis 7 Halogenatomen, C₁-C₃-Alkoxy, C₁-C₃-Halogenalkoxy mit 1 bis 7 Halogenatomen, C₁-C₃-Alkylthio, C₁-C₃-Halogenalkylthio mit 1 bis 7 Halogenatomen, Halogen, Cyano oder Nitro substituiertes 5-Thiazolyl steht oder 3-Pyridyl steht,
R⁴ für C₁-C₁₀-Alkyl, C₃-C₆-Cycloalkyl, durch 1 bis 6 Reste aus der Gruppe Halogen, C₁-C₄-Alkoxy, C₁-C₄-Halogenalkoxy mit 1 bis 9 Halogenatomen substituiertes C₁-C₈-Alkyl, durch 1 oder 2 Reste aus der Reihe Methyl, Ethyl, Fluor und Chlor substituiertes C₃-C₆-Cycloalkyl, Phenyl, Benzyl, oder durch 1 bis 3 Ringsubstituenten aus der Gruppe Halogen, C₁-C₄-Alkyl, C₁-C₄-Halogenalkyl mit 1 bis 9 Halogenatomen, C₁-C₄-Alkoxy, C₁-C₄-Halogenalkoxy mit 1 bis 9 Halogenatomen, C₁-C₄-Alkylthio, Nitro oder Cyano substituiertes Phenyl, Benzyl oder Heterocyclylmethyl steht, wobei Heterocyclyl für einen ungesättigten oder gesättigten 5- oder 6-gliedrigen Heterocyclus mit einem Heteroatomen aus der Reihe Stickstoff, Sauerstoff, Schwefel wie z.B. Furan, Tetrahydrofuran, Thiophen oder Pyridin steht,
Het flir jeweils unsubstituiertes oder einfach oder zweifach (insbesondere einfach) substituiertes Thiazolyl, Pyridyl oder Tetrahydrofuranyl steht, wobei die Substituenten aus der Reihe Fluor, Chlor, Methyl und Methoxy ausgewählt sind,
R⁵ für C₁-C₄-Alkyl, C₃-C₆-Cycloalkyl, Benzyl oder Phenylethyl steht, jeweils gegebenenfalls einfach bis fünffach substituiert, wobei als Substituenten NH₂, OH, Cl, Br, F, C₁-C₄-Alkoxy, C₁-C₄-Alkylthio, C₁-C₄-Alkyl und Aryl, insbesondere Phenyl in Frage kommen, oder für Heterocyclylmethyl steht, wobei Heterocyclyl flir einen ungesättigten oder gesättigten 5- oder 6-gliedrigen Heterocyclus mit einem Heteroatomen aus der Reihe Stickstoff, Sauerstoff, Schwefel wie z.B. Furan, Tetrahydrofuran, Thiophen oder Pyridin steht.

4. Verfahren gemäß Anspruch 1, wobei in den Formeln (I), (II) und (III)
R¹ für Wasserstoff, Methyl oder Ethyl, hervorgehoben für Wasserstoff steht,
R² für Wasserstoff, Methyl, Ethyl, n-Propyl, Cyclopropyl, Cyclopentyl, Allyl, Propargyl oder p-Chlorbenzyl steht,
R⁴ für Methyl, Ethyl, n-Propyl, Cyclopropyl, Cyclopentyl, Cyclohexyl, Phenyl, Benzyl oder Furfuryl steht,
Het für einen der Reste 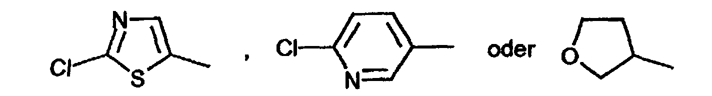 steht, und
R⁵ für den jeweiligen Rest R⁴ der umzusetzenden Verbindung der Formel (II) oder die Reste Benzyl, NH₂-CH₂-CH₂-, HO-CH₂-CH₂-, n-Hexyl oder Cyclohexyl steht.

5. Verfahren gemäß Anspruch 1 zur Herstellung einer Verbindung der Formel 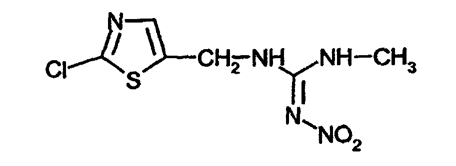 **dadurch gekennzeichnet, dass** man eine Verbindung der Formel (IIa) 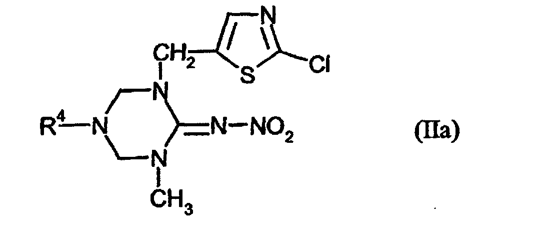 gegebenenfalls in Gegenwart eines Verdünnungsmittels mit einer Verbindung der Formel (III) 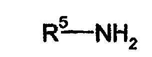 wobei R⁴ = R⁵
für Methyl, Ethyl, Cyclopropyl, Cyclopentyl, Benzyl oder Furfuryl steht, umsetzt.

6. Verfahren gemäß Anspruch 1 zur Herstellung Verbindung einer der Formel 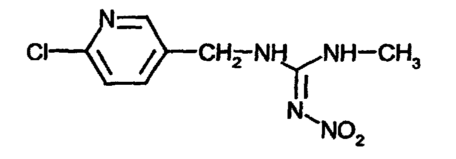 **dadurch gekennzeichnet**, man dass eine Verbindung der Formel (IIb) 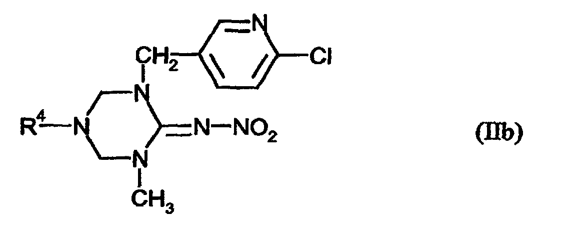 gegebenenfalls in Gegenwart eines Verdünnungsmittels mit einer Verbindung der Formel (III) 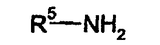 wobei R⁴ = R⁵
für Methyl, Ethyl, Cyclopropyl, Cyclopentyl, Benzyl oder Furfuryl steht, umsetzt.

7. Verfahren gemäß Anspruch 1 zur Herstellung einer Verbindung der Formel 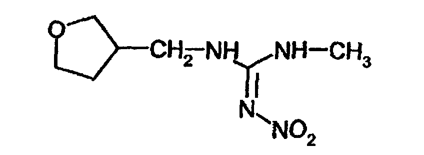 **dadurch gekennzeichnet**, man dass eine Verbindung der Formel (IIc) 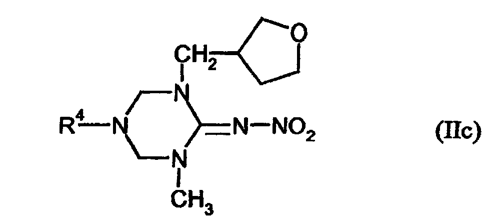 gegebenenfalls in Gegenwart eines Verdünnungsmittels mit einer Verbindung der Formel (III) 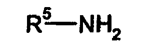 wobei R⁴ = R⁵
für Methyl, Ethyl, Cyclopropyl, Cyclopentyl, Benzyl oder Furfuryl steht, umsetzt.

8. Verfahren gemäß Anspruch 1, **dadurch gekennzeichnet, dass** die Umsetzung in Abwesenheit eines Verdünnungsmittels durchgeführt wird.

9. Verfahren gemäß Anspruch 1, **dadurch gekennzeichnet, dass** die Umsetzung in Gegenwart eines Verdünnungsmittels aus der Reihe der Alkohole durchgeführt wird.

10. Verfahren gemäß Anspruch 1, **dadurch gekennzeichnet, dass** es bei einer Temperatur zwischen 0°C und 200°C durchgeführt wird.

## Claims

1. Process for the preparation of compounds of the formula (I) 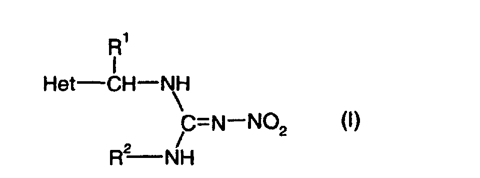 in which
R¹ is hydrogen or C₁-C₄-alkyl,
R² is hydrogen, C₁-C₆-alkyl, C₃-C₆-cycloalkyl or a radical -CH₂R³,
R³ is C₂-C₅-alkenyl, C₂-C₅-alkinyl, phenyl, cyanophenyl, nitrophenyl, halogenophenyl having from 1 to 3 halogen atoms, phenyl substituted by C₁-C₃-alkyl, C₁-C₃-halogenoalkyl having from 1 to 7 halogen atoms, C₁-C₃-alkoxy or C₁-C₃-halogenoalkoxy having from 1 to 7 halogen atoms, 3-pyridyl, 5-thiazolyl, 5-thiazolyl substituted by one to two (preferably one) substituents from the group consisting of C₁-C₃-alkyl, C₁-C₃-halogenoalkyl having from 1 to 7 halogen atoms, cyclopropyl, halogenocyclopropyl, C₂-C₃alkenyl, C₂-C₃-alkinyl, C₁-C₃-alkoxy, C₂-C₃-halogenoalkenyl having from 1 to 4 halogen atoms, C₂-C₃-halogenoalkinyl having from 1 to 3 halogen atoms, C₁-C₃-halogenoalkoxy having from 1 to 7 halogen atoms, C₁-C₃-alkylthio, C₁-C₃-halogenoalkylthio having from 1 to 7 halogen atoms, allyloxy, propargyloxy, allylthio, propargylthio, halogenoallyloxy, halogenoallylthio, halogen, cyano or nitro; or 3-pyridyl substituted by one to four (preferably one or two) radicals from the group consisting of C₁-C₃-halogenoalkyl having from 1 to 7 halogen atoms, cyclopropyl, halogenocyclopropyl, C₂-C₃-alkenyl, C₂-C₃-alkinyl, C₂-C₃-halogenoalkenyl having from 1 to 4 halogen atoms, C₂-C₃-halogenoalkinyl having from 1 to 3 halogen atoms, C₁-C₃-halogenoalkoxy having from 1 to 7 halogen atoms, C₁-C₃-alkylthio, C₁-C₃-halogenoalkylthio having from 1 to 7 halogen atoms, allyloxy, propargyloxy, allylthio, propargylthio, halogenoallyloxy, halogenoallylthio, cyano, nitro, C₁-C₃-alkyl, C₁-C₃-alkoxy or halogen,
Het is an unsubstituted or substituted aromatic or non-aromatic, monocyclic or bicyclic heterocyclic radical, preferably from the series 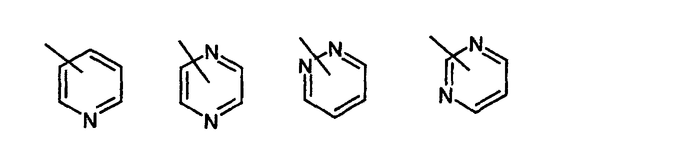 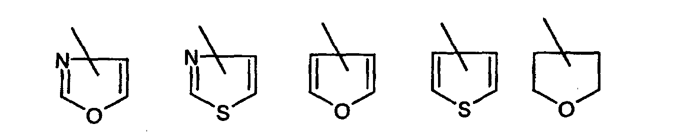 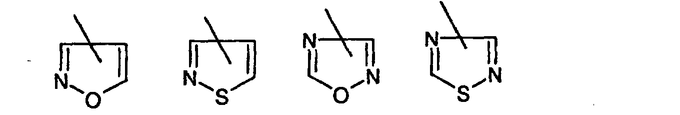 which may include one to two substituents from the group consisting of C₁-C₃-halogenoalkyl having from 1 to 7 halogen atoms, cyclopropyl, halogenocyclopropyl having from 1 to 3 halogen atoms, C₂-C₃-alkenyl, C₂-C₃-alkinyl, C₂-C₃-halogenoalkenyl having from 1 to 4 halogen atoms, C₂-C₃-halogenoalkinyl having from 1 to 3 halogen atoms, C₁-C₃-halogenoalkoxy having from 1 to 7 halogen atoms, C₁-C₃-alkylthio, C₁-C₃-halogenoalkylthio having from 1 to 7 halogen atoms, allyloxy, propargyloxy, allylthio, propargylthio, halogenoallyloxy, halogenoallylthio, cyano, nitro, C₁-C₃-alkyl, C₁-C₃-alkoxy and halogen,
**characterized in that** a compound of the formula (II) 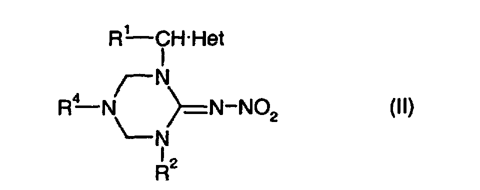 in which
R¹, R² and Het are as defined above, and
R⁴ is C₁-C₁₀-alkyl, C₃-C₆-cycloalkyl, phenyl, arylalkyl or heterocyclylmethyl, each of which may be unsubstituted or substituted, where heterocyclyl is an unsaturated or saturated 5- or 6-membered heterocycle containing one or more heteroatoms from the series nitrogen, oxygen, sulphur, such as, for example, furan, tetrahydrofuran, thiophene or pyridine,
optionally in the presence of a diluent, is reacted with compounds of the formula (III),
R⁵-NH₂ (III)
in which
R⁵ is optionally substituted alkyl, cycloalkyl, aryl, arylalkyl or heterocyclylmethyl, where heterocyclyl is an unsaturated or saturated 5- or 6-membered heterocycle containing one or more heteroatoms from the series nitrogen, oxygen, sulphur, such as, for example, furan, tetrahydrofuran, thiophene or pyridine,
where the compounds of the formula (III) are used in a molar ratio of from 0.5:1 to 10:1, based on the starting compound of the formula (II).

2. Process according to Claim 1, where in the formulae (I), (II) and (III) according to Claim 1,
R² is hydrogen, methyl, ethyl, n-propyl, i-propyl or n-butyl, cyclopropyl, cyclopentyl, cyclohexyl or a radical -CH₂R³,
R³ is C₂-C₅-alkenyl, C₂-C₅-alkinyl, phenyl, cyanophenyl, nitrophenyl, halogenophenyl having from 1 to 3 halogen atoms, phenyl substituted by C₁-C₃-alkyl, C₁-C₃-halogenoalkyl having from 1 to 7 halogen atoms, C₁-C₃-alkoxy or C₁-C₃-halogenoalkoxy having from 1 to 7 halogen atoms, 3-pyridyl, 5-thiazolyl, 5-thiazolyl substituted by one to two (preferably one) substituents from the group consisting of C₁-C₃-alkyl, C₁-C₃-halogenoalkyl having from 1 to 7 halogen atoms, cyclopropyl, halogenocyclopropyl, C₂-C₃-alkenyl, C₂-C₃-alkinyl, C₁-C₃-alkoxy, C₂-C₃-halogenoalkenyl having from 1 to 4 halogen atoms, C₂-C₃-halogenoalkinyl having from 1 to 3 halogen atoms, C₁-C₃-halogenoalkoxy having from 1 to 7 halogen atoms, C₁-C₃-alkylthio, C₁-C₃-halogenoalkylthio having from 1 to 7 halogen atoms, allyloxy, propargyloxy, allylthio, propargylthio, halogenoallyloxy, halogenoallylthio, halogen, cyano or nitro; or 3-pyridyl substituted by one to two (preferably one) radicals from the group consisting of C₁-C₃-halogenoalkyl having from 1 to 7 halogen atoms, cyclopropyl, halogenocyclopropyl, C₂-C₃-alkenyl, C₂-C₃-alkinyl, C₂-C₃-halogenoalkenyl having from 1 to 4 halogen atoms, C₂-C₃-halogenoalkinyl having from 1 to 3 halogen atoms, C₁-C₃-halogenoalkoxy having from 1 to 7 halogen atoms, C₁-C₃-alkylthio, C₁-C₃-halogenoalkylthio having from 1 to 7 halogen atoms, allyloxy, propargyloxy, allylthio, propargylthio, halogenoallyloxy, halogenoallylthio, cyano, nitro, C₁-C₃-alkyl, C₁-C₃-alkoxy or halogen,
R⁴ is C₁-C₁₀-alkyl, C₃-C₆-cycloalkyl, C₁-C₁₀-alkyl substituted by from I to 6 radicals from the group consisting of halogen, hydroxyl, C₁-C₄-alkoxy, C₁-C₄-halogenoalkoxy having from 1 to 9 halogen atoms, di-(C₁-C₄-alkyl)-amino or C₁-C₅-alkoxycarbonyl, C₃-C₆-cycloalkyl substituted by from 1 to 4 radicals from the series C₁-C₄-alkyl or halogen, phenyl, benzyl, or phenyl or benzyl substituted by from 1 to 3 ring substituents from the group consisting of halogen, C₁-C₄-alkyl, C₁-C₄-halogenoalkyl having from 1 to 9 halogen atoms, C₁-C₄-alkoxy, C₁-C₄-halogenoalkoxy having from 1 to 9 halogen atoms, C₁-C₄-alkylthio, nitro or cyano, or heterocyclylmethyl, where heterocyclyl is an unsaturated or saturated 5- or 6-membered heterocycle having one or two (preferably one) heteroatoms from the series nitrogen, oxygen, sulphur, such as, for example, furan, tetrahydrofuran, thiophene or pyridine,
Het is an unsubstituted or mono- or disubstituted (preferably monosubstituted) heterocyclic radical from the series 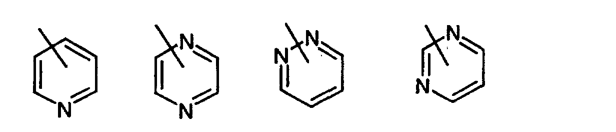 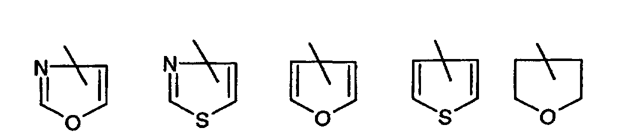 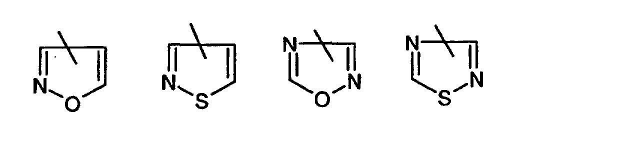 in particular 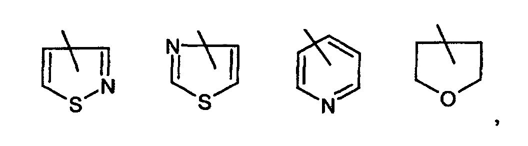 the substituents being chosen from the series fluorine, chlorine, bromine, methyl, ethyl, methoxy and ethoxy,
R⁵ is C₁-C₁₀-alkyl, C₃-C₁₀-cycloalkyl, aryl-C₁-C₄-alkyl, in particular phenyl-C₁-C₄-alkyl, each of which may be mono- or polysubstituted, suitable substituents being NH₂, OH, SH, halogen, C₁-C₆-alkoxy, C₁-C₆-alkylthio, C₁-C₆-alkyl and aryl, in particular phenyl, or R⁵ is heterocyclylmethyl, where heterocyclyl is an unsaturated or saturated 5- or 6-membered heterocycle containing one or two (preferably one) heteroatoms from the series nitrogen, oxygen, sulphur, such as, for example, furan, tetrahydrofuran, thiophene or pyridine.

3. Process according to Claim 1, where, in the formulae (I), (II) and (III) according to Claim 1,
R¹ is hydrogen, methyl or ethyl,
R² is hydrogen, methyl, ethyl, n-propyl, i-propyl, n-butyl, cyclopropyl, cyclopentyl, cyclohexyl or a radical -CH₂R³,
R³ is C₂-C₃-alkenyl, C₂-C₃-alkinyl, phenyl, cyanophenyl, nitrophenyl, halogenophenyl having from 1 to 3 halogen atoms, phenyl substituted by C₁-C₃-alkyl, C₁-C₃-halogenoalkyl having from 1 to 7 halogen atoms, C₁-C₃-alkoxy or C₁-C₃-halogenoalkoxy having from 1 to 7 halogen atoms, 3-pyridyl, 5-thiazolyl, 5-thiazolyl substituted by one or two (preferably one) substituents from the group consisting of C₁-C₃-alkyl, C₁-C₃-halogenoalkyl having from I to 7 halogen atoms, C₁-C₃-alkoxy, C₁-C₃-halogenoalkoxy having from 1 to 7 halogen atoms, C₁-C₃-alkylthio, C₁-C₃-halogenoalkylthio having from 1 to 7 halogen atoms, halogen, cyano or nitro, or is 3-pyridyl,
R⁴ is C₁-C₁₀-alkyl, C₃-C₆-cycloalkyl, C₁-C₈-alkyl, substituted by from 1 to 6 radicals from the group consisting of halogen, C₁-C₄-alkoxy, C₁-C₄-halogenoalkoxy having from 1 to 9 halogen atoms, C₃-C₆-cycloalkyl substituted by 1 or 2 radicals from the series methyl, ethyl, fluorine and chlorine, phenyl, benzyl, or phenyl or benzyl substituted by from I to 3 ring substituents from the group consisting of halogen, C₁-C₄-alkyl, C₁-C₄-halogenoalkyl having from I to 9 halogen atoms, C₁-C₄-alkoxy, C₁-C₄-halogenoalkoxy having from 1 to 9 halogen atoms, C₁-C₄-alkylthio, nitro or cyano, or heterocyclyl-methyl, where heterocyclyl is an unsaturated or saturated 5- or 6-membered heterocycle containing one heteroatom from the series nitrogen, oxygen, sulphur, such as, for example, furan, tetrahydrofuran, thiophene or pyridine,
Het is thiazolyl, pyridyl or tetrahydrofuranyl, each of which may be unsubstituted or mono- or disubstituted (in particular monosubstituted), the substituents being chosen from the series fluorine, chlorine, methyl and methoxy,
R⁵ is C₁-C₄-alkyl, C₃-C₆-cycloalkyl, benzyl or phenylethyl, each of which may be substituted from one to five times, suitable substituents being NH₂, OH, Cl, Br, F, C₁-C₄-alkoxy, C₁-C₄-alkylthio, C₁-C₄-alkyl and aryl, in particular phenyl, or R⁵ is heterocyclylmethyl, where heterocyclyl is an unsaturated or saturated 5- or 6-membered heterocycle containing a heteroatom from the series nitrogen, oxygen, sulphur, such as, for example, furan, tetrahydrofuran, thiophene or pyridine.

4. Process according to Claim 1, where, in the formulae (I), (II) and (III),
R¹ is hydrogen, methyl or ethyl, especially hydrogen,
R² is hydrogen, methyl, ethyl, n-propyl, cyclopropyl, cyclopentyl, allyl, propargyl or p-chlorobenzyl,
R⁴ is methyl, ethyl, n-propyl, cyclopropyl, cyclopentyl, cyclohexyl, phenyl, benzyl or furfuryl,
Het is one of the radicals 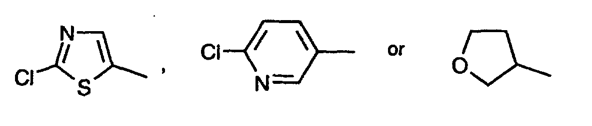 and
R⁵ is the respective radical R⁴ of the compound of the formula (II) to be reacted or the radicals benzyl, NH₂-CH₂-CH₂-, HO-CH₂-CH₂-, n-hexyl or cyclohexyl.

5. Process according to Claim 1 for the preparation of a compound of the formula 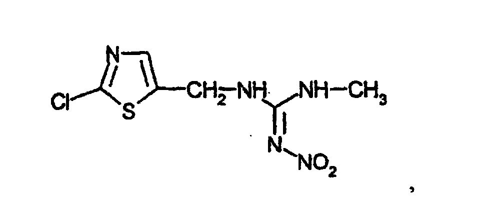 **characterized in that** a compound of the formula (IIa) 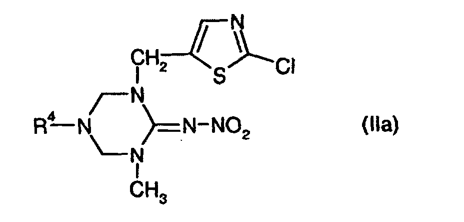 optionally in the presence of a diluent, is reacted with a compound of the formula (III)
R⁵-NH₂
where R⁴ = R⁵ and
is methyl, ethyl, cyclopropyl, cyclopentyl, benzyl or furfuryl.

6. Process according to Claim 1 for the preparation of a compound of the formula 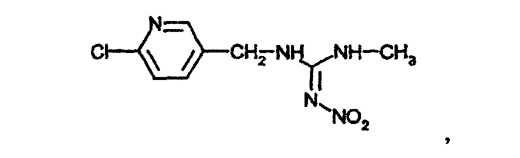 **characterized in that** a compound of the formula (IIb) 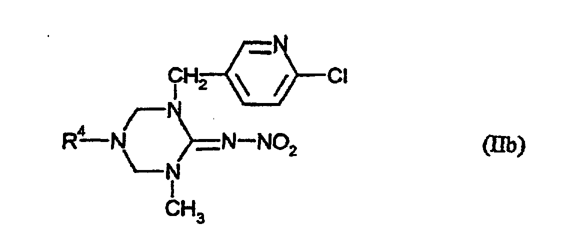 optionally in the presence of a diluent, is reacted with a compound of the formula (III)
R⁵-NH₂
where R⁴ = R⁵ and
is methyl, ethyl, cyclopropyl, cyclopentyl, benzyl or furfuryl.

7. Process according to Claim 1 for the preparation of a compound of the formula 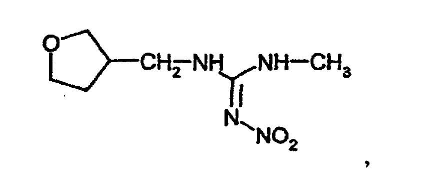 **characterized in that** a compound of the formula (IIc) 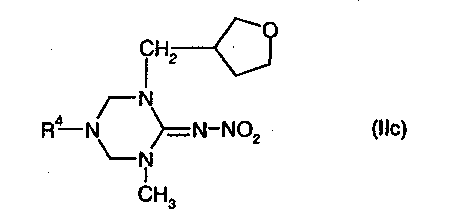 optionally in the presence of a diluent, is reacted with a compound of the formula (III)
R⁵-NH₂
where R⁴ = R⁵ and
is methyl, ethyl, cyclopropyl, cyclopentyl, benzyl or furfuryl.

8. Process according to Claim 1, **characterized in that** the reaction is carried out in the absence of a diluent.

9. Process according to Claim 1, **characterized in that** the reaction is carried out in the presence of a diluent from the series of alcohols.

10. Process according to Claim 1, **characterized in that** it is carried out at a temperature between 0°C and 200°C.

## Revendications

1. Procédé de préparation de composés de la formule (I) : 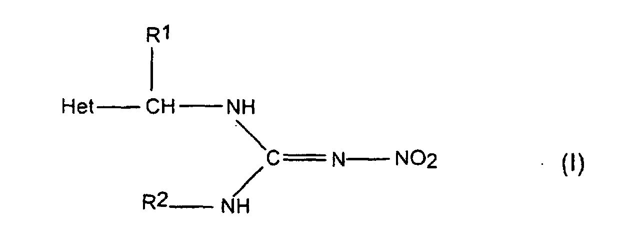 où
R¹ représente l'atome d'hydrogène ou un radical alcoyle en C₁-C₄ ;
R² représente l'atome d'hydrogène ou un radical alcoyle en C₁-C₆, cycloalcoyle en C₃-C₆ ou un reste -CH₂R³ ;
R³ représente un radical alcényle en C₂-C₅, alcynyle en C₂-C₅, le radical phényle, cyanophényle, nitrophényle, un radical halogénophényle ayant 1 à 3 atomes d'halogène, un radical phényle substitué par un radical alcoyle en C₁-C₃, halogénoalcoyle en C₁-C₃ ayant 1 à 7 atomes d'halogène, alcoxy en C₁-C₃ ou halogénoalcoxy en C₁-C₃ ayant 1 à 7 atomes d'halogène, le radical 3-pyridyle, 5-thiazolyle, un radical 5-thiazolyle substitué par un ou deux (de préférence, un) substituants du groupe des radicaux alcoyle en C₁-C₃, halogénoalcoyle en C₁-C₃ ayant 1 à 7 atomes d'halogène, cyclopropyle, halogénocyclopropyle, alcényle en C₂-C₃, alcynyle en C₂-C₃, alcoxy en C₁-C₃, halogénoalcényle en C₂-C₃ ayant 1 à 4 atomes d'halogène, halogénoalcynyle en C₂-C₃ ayant 1 à 3 atomes d'halogène, halogénoalcoxy en C₁-C₃ ayant 1 à 7 atomes d'halogène, alcoylthio en C₁-C₃, halogénoalcoylthio en C₁-C₃ ayant 1 à 7 atomes d'halogène, allyloxy, propargyloxy, allylthio, propargylthio, halogénoallyloxy, halogénoallylthio, un atome d'halogène, le radical cyano ou nitro ; ou représente un radical 3-pyridyle substitué par un à quatre (de préférence, un ou deux) substituants du groupe des radicaux halogénoalcoyle en C₁-C₃ ayant 1 à 7 atomes d'halogène, cyclopropyle, halogénocyclopropyle, alcényle en C₂-C₃, alcynyle en C₂-C₃, halogénoalcényle en C₂-C₃ ayant 1 à 4 atomes d'halogène, halogénoalcynyle en C₂-C₃ ayant 1 à 3 atomes d'halogène, halogénoalcoxy en C₁-C₃ ayant 1 à 7 atomes d'halogène, alcoylthio en C₁-C₃, halogénoalcoylthio en C₁-C₃ ayant 1 à 7 atomes d'halogène, allyloxy, propargyloxy, allylthio, propargylthio, halogénoallyloxy, halogénoallylthio, le radical cyano ou nitro, un radical alcoyle en C₁-C₃, alcoxy en C₁-C₃ ou un atome d'halogène ;
Het représente un reste hétérocyclique monocyclique ou bicyclique, aromatique ou non aromatique, substitué ou non substitué, de préférence de la série : 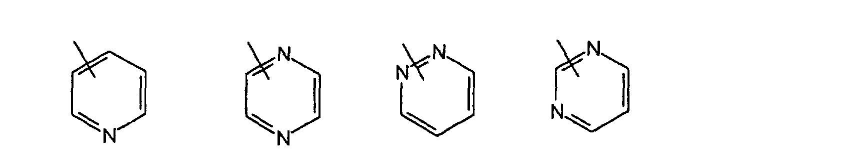 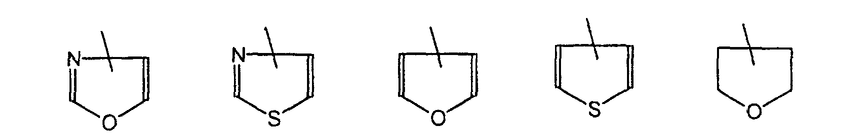 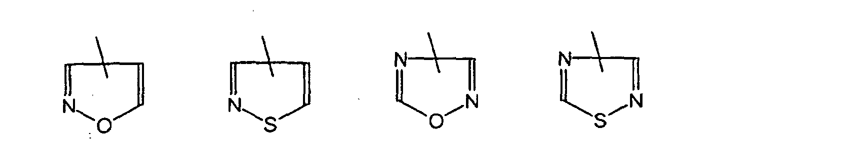 qui peuvent contenir un ou deux substituants du groupe des radicaux halogénoalcoyle en C₁-C₃ ayant 1 à 7 atomes d'halogène, cyclopropyle, halogénocyclopropyle ayant 1 à 3 atomes d'halogène, alcényle en C₂-C₃, alcynyle en C₂-C₃, halogénoalcényle en C₂-C₃ ayant 1 à 4 atomes d'halogène, halogénoalcynyle en C₂-C₃ ayant 1 à 3 atomes d'halogène, halogénoalcoxy en C₁-C₃ ayant 1 à 7 atomes d'halogène, alcoylthio en C₁-C₃, halogénoalcoylthio en C₁-C₃ ayant 1 à 7 atomes d'halogène, allyloxy, propargyloxy, allylthio, propargylthio, halogénoallyloxy, halogénoallylthio, le radical cyano ou nitro, un radical alcoyle en C₁-C₃, alcoxy en C₁-C₃ ou un atome d'halogène,
**caractérisé en ce que** l'on fait réagir un composé de la formule (II) : 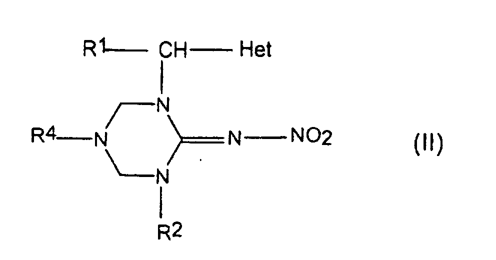 dans laquelle :
R¹, R² et Het ont les significations indiquées ci-dessus, et
R⁴ représente un radical alcoyle en C₁-C₁₀, cycloalcoyle en C₃-C₆, phényle, arylalcoyle ou hétérocyclylméthyle, chaque fois substitué ou non substitué, où le reste hétérocyclyle représente un hétérocycle à 5 ou 6 membres, saturé ou insaturé, avec un ou plusieurs hétéroatomes de la série de l'azote, l'oxygène, le soufre comme par exemple furanne, tétrahydrofuranne, thiophène ou pyridine,
le cas échéant en présence d'un agent de dilution, avec des composés de la formule (III) :
R⁵-NH₂ (III)
dans laquelle:
R⁵ représente un radical alcoyle, cycloalcoyle, aryle, arylalcoyle ou hétérocyclylméthyle, chaque fois substitué ou non substitué, où le reste hétérocyclyle représente un hétérocycle à 5 ou 6 membres, saturé ou insaturé, avec un ou plusieurs hétéroatomes de la série de l'azote, l'oxygène, le soufre comme par exemple furanne, tétrahydrofuranne, thiophène ou pyridine,
où les composés de la formule (III) sont mis en oeuvre en un rapport molaire allant de 0,5:1 à 10:1, sur base du composé de départ de la formule (II).

2. Procédé selon la revendication 1, où dans les formules (I), (II) et (III) selon la revendication 1,
R¹ représente l'atome d'hydrogène, le radical méthyle, éthyle, n- ou i-propyle ;
R² représente l'atome d'hydrogène, le radical méthyle, éthyle, n-propyle, i-propyle ou n-butyle, cyclopropyle, cyclopentyle, cyclohexyle ou un reste -CH₂R³ ;
R³ représente un radical alcényle en C₂-C₅, alcynyle en C₂-C₅, le radical phényle, cyanophényle, nitrophényle, un radical halogénophényle ayant 1 à 3 atomes d'halogène, un radical phényle substitué par un radical alcoyle en C₁-C₃, halogénoalcoyle en C₁-C₃ ayant 1 à 7 atomes d'halogène, alcoxy en C₁-C₃ ou halogénoalcoxy en C₁-C₃ ayant 1 à 7 atomes d'halogène, représente le radical 3-pyridyle, 5-thiazolyle, un radical 5-thiazolyle substitué par un ou deux (de préférence, un) substituants du groupe des radicaux alcoyle en C₁-C₃, halogénoalcoyle en C₁-C₃ ayant 1 à 7 atomes d'halogène, cyclopropyle, halogénocyclopropyle, alcényle en C₂-C₃, alcynyle en C₂-C₃, alcoxy en C₁-C₃, halogénoalcényle en C₂-C₃ ayant 1 à 4 atomes d'halogène, halogénoalcynyle en C₂-C₃ ayant 1 à 3 atomes d'halogène, halogénoalcoxy en C₁-C₃ ayant 1 à 7 atomes d'halogène, alcoylthio en C₁-C₃, halogénoalcoylthio en C₁-C₃ ayant 1 à 7 atomes d'halogène, allyloxy, propargyloxy, allylthio, propargylthio, halogénoallyloxy, halogénoallylthio, un atome d'halogène, le radical cyano ou nitro ; ou représente un radical 3-pyridyle substitué par un ou deux (de préférence, un) substituants du groupe des radicaux halogénoalcoyle en C₁-C₃ ayant 1 à 7 atomes d'halogène, cyclopropyle, halogénocyclopropyle, alcényle en C₂-C₃, alcynyle en C₂-C₃, halogénoalcényle en C₂-C₃ ayant 1 à 4 atomes d'halogène, halogénoalcynyle en C₂-C₃ ayant 1 à 3 atomes d'halogène, halogénoalcoxy en C₁-C₃ ayant 1 à 7 atomes d'halogène, alcoylthio en C₁-C₃, halogénoalcoylthio en C₁-C₃ ayant 1 à 7 atomes d'halogène, allyloxy, propargyloxy, allylthio, propargylthio, halogénoallyloxy, halogénoallylthio, le radical cyano ou nitro, un radical alcoyle en C₁-C₃, alcoxy en C₁-C₃ ou un atome d'halogène ;
R⁴ représente un radical alcoyle en C₁-C₁₀, cycloalcoyle en C₃-C₆, le radical alcoyle en C₁-C₁₀ substitué par 1 à 6 restes du groupe des atomes d'halogène, du radical hydroxyle, des radicaux alcoxy en C₁-C₄, halogénoalcoxy en C₁-C₄ ayant 1 à 9 atomes d'halogène, di(alcoyle en C₁-C₄)amino ou (alcoxy en C₁-C₅)carbonyle, le radical cycloalcoyle en C₃-C₆ substitué par 1 à 4 restes de la série des radicaux alcoyle en C₁-C₄ ou des atomes d'halogène, représente le radical phényle, benzyle ou le radical phényle ou benzyle substitué par 1 à 3 substituants cycliques du groupe des atomes d'halogène, des radicaux alcoyle en C₁-C₄, halogénoalcoyle en C₁-C₄ ayant 1 à 9 atomes d'halogène, alcoxy en C₁-C₄, halogénoalcoxy en C₁-C₄ ayant 1 à 9 atomes d'halogène, alcoylthio en C₁-C₄, le radical nitro ou cyano, ou représente un radical hétérocyclylméthyle, où le reste hétérocyclyle représente un hétérocycle à 5 ou 6 membres, saturé ou insaturé, avec un ou deux (de préférence, un) hétéroatomes de la série de l'azote, l'oxygène, le soufre comme par exemple furanne, tétrahydrofuranne, thiophène ou pyridine ;
Het représente un reste hétérocyclique non substitué ou substitué une ou deux fois (de préférence, une fois), de la série : 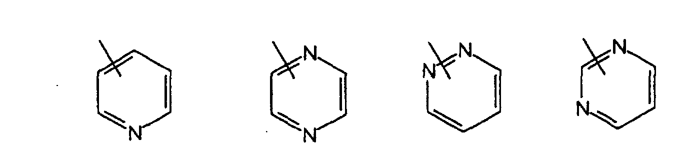 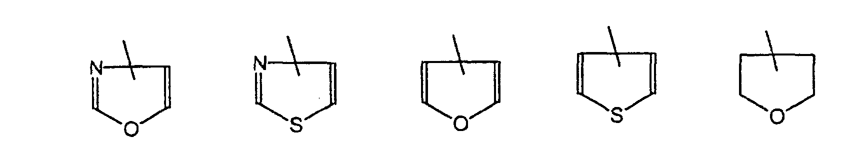 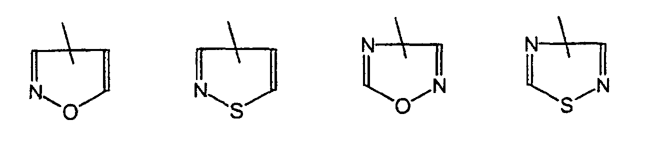 en particulier : 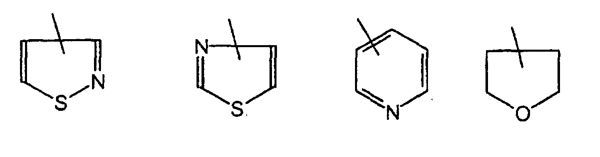 où les substituants sont choisis parmi la série des atomes de fluor, de chlore, de brome, des radicaux méthyle, éthyle, méthoxy et éthoxy ;
R⁵ représente un radical alcoyle en C₁-C₁₀, cycloalcoyle en C₃-C₁₀, aryl(alcoyle en C₁-C₄), en particulier phényl(alcoyle en C₁-C₄), chaque fois le cas échéant substitué une ou plusieurs fois, où comme substituant on envisage NH₂, OH, SH, un atome d'halogène, alcoxy en C₁-C₆, alcoylthio en C₁-C₆, alcoyle en C₁-C₆, et aryle, en particulier le radical phényle, ou représente un radical hétérocyclylméthyle, où le reste hétérocyclyle représente un hétérocycle à 5 ou 6 membres, saturé ou insaturé, avec un ou deux (de préférence, un) hétéroatomes de la série des atomes d'azote, d'oxygène, de soufre comme par exemple furanne, tétrahydrofuranne, thiophène ou pyridine.

3. Procédé selon la revendication 1, où dans les formules (I), (II) et (III) selon la revendication 1,
R¹ représente l'atome d'hydrogène, le radical méthyle ou éthyle ;
R² représente l'atome d'hydrogène, le radical méthyle, éthyle, n-propyle, i-propyle, n-butyle, cyclopropyle, cyclopentyle, cyclohexyle ou un reste -CH₂R³ ;
R³ représente un radical alcényle en C₂-C₃, alcynyle en C₂-C₃, le radical phényle, cyanophényle, nitrophényle, un radical halogénophényle ayant 1 à 3 atomes d'halogène, un radical phényle substitué par un radical alcoyle en C₁-C₃, halogénoalcoyle en C₁-C₃ ayant 1 à 7 atomes d'halogène, alcoxy en C₁-C₃ ou halogénoalcoxy en C₁-C₃ ayant 1 à 7 atomes d'halogène, représente le radical 3-pyridyle, 5-thiazolyle, un radical 5-thiazolyle ou 3-pyridyle chaque fois substitué par un ou deux (de préférence, un) substituants du groupe des radicaux alcoyle en C₁-C₃, halogénoalcoyle en C₁-C₃ ayant 1 à 7 atomes d'halogène, alcoxy en C₁-C₃, halogénoalcoxy en C₁-C₃ ayant 1 à 4 atomes d'halogène, alcoylthio en C₁-C₃, halogénoalcoylthio en C₁-C₃ ayant 1 à 7 atomes d'halogène, un atome d'halogène, le radical cyano ou nitro ;
R⁴ représente un radical alcoyle en C₁-C₁₀, cycloalcoyle en C₃-C₆, le radical alcoyle en C₁-C₈ substitué par 1 à 6 restes du groupe des atomes d'halogène, des radicaux alcoxy en C₁-C₄ et halogénoalcoxy en C₁-C₄ ayant 1 à 9 atomes d'halogène, représente le radical cycloalcoyle en C₃-C₆ substitué par 1 ou 2 restes de la série des radicaux méthyle, éthyle, des atomes de fluor et de chlore, ou représente le radical phényle ou benzyle substitué par 1 à 3 substituants cycliques du groupe des atomes d'halogène, des radicaux alcoyle en C₁-C₄, halogénoalcoyle en C₁-C₄ ayant 1 à 9 atomes d'halogène, alcoxy en C₁-C₄, halogénoalcoxy en C₁-C₄ ayant 1 à 9 atomes d'halogène, alcoylthio en C₁-C₄, le radical nitro ou cyano, ou représente un radical hétérocyclylméthyle, où le reste hétérocyclyle représente un hétérocycle à 5 ou 6 membres, saturé ou insaturé, avec un hétéroatome de la série de l'atome d'azote, d'oxygène, de soufre comme par exemple furanne, tétrahydrofuranne, thiophène ou pyridine.
Het représente le radical thiazolyle, pyridyle ou tétrahydrofurannyle, chaque fois non substitué ou substitué une ou deux fois (en particulier, une fois), où les substiuants sont choisis parmi la série des atomes de fluor, de chlore, des radicaux méthyle et méthoxy ;
R⁵ représente un radical alcoyle en C₁-C₄, cycloalcoyle en C₃-C₆, le radical benzyle ou phényléthyle, chaque fois le cas échéant substitué une à cinq fois, où comme substituant on envisage NH₂, OH, Cl, Br, F, un radical alcoxy en C₁-C₄, alcoylthio en C₁-C₄, alcoyle en C₁-C₄, et aryle, en particulier le radical phényle, ou représente un radical hétérocyclylméthyle, où le reste hétérocyclyle représente un hétérocycle à 5 ou 6 membres, saturé ou insaturé, avec un hétéroatome de la série des atomes d'azote, d'oxygène, de soufre comme par exemple furanne, tétrahydrofuranne, thiophène ou pyridine.

4. Procédé suivant la revendication 1, où dans les formules (I), (II) et (III) :
R¹ représente l'atome d'hydrogène, le radical méthyle ou éthyle, avec insistance, l'atome d'hydrogène ;
R² représente l'atome d'hydrogène, le radical méthyle, éthyle, n-propyle, cyclopropyle, cyclopentyle, allyle, propargyle ou p-chlorobenzyle ;
R⁴ représente le radical méthyle, éthyle, n-propyle, cyclopropyle, cyclopentyle, cyclohexyle, phényle, benzyle ou furfurylméthyle ;
Het représente un des restes : 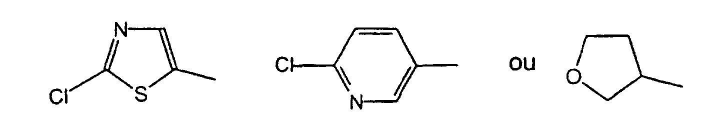
R⁵ représente les restes R⁴ du composé de formule (II) à faire réagir, ou les restes benzyle, NH₂-CH₂-CH₂-, HO-CH₂-CH₂-, n-hexyle ou cyclohexyle.

5. Procédé selon la revendication 1, pour la préparation d'un composé de la formule : 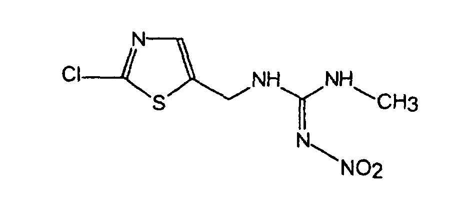 **caractérisé en ce que** l'on fait réagir un composé de la formule (IIa) : 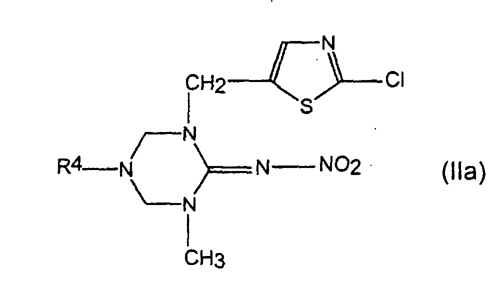 le cas échéant, en présence d'un agent de dilution avec un composé de la formule (III) :
R⁵ -NH₂
où R⁴ = R⁵ représente le radical méthyle, éthyle, cyclopropyle, cyclopentyle, benzyle ou furfuryle.

6. Procédé selon la revendication 1, pour la préparation d'un composé de la formule : 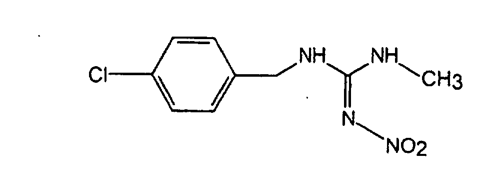 **caractérisé en ce que** l'on fait réagir un composé de la formule (IIb) : 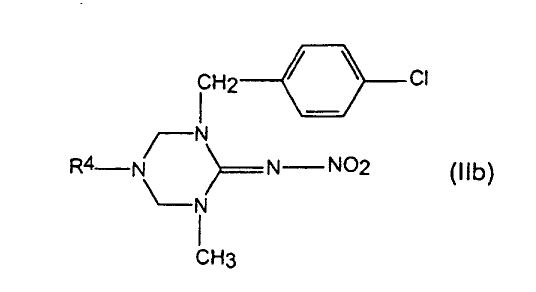 le cas échéant, en présence d'un agent de dilution avec un composé de la formule (III) :
R⁵ -NH₂
où R⁴ = R⁵ représente le radical méthyle, éthyle, cyclopropyle, cyclopentyle, benzyle ou furfuryle.

7. Procédé selon la revendication 1, pour la préparation d'un composé de la formule : 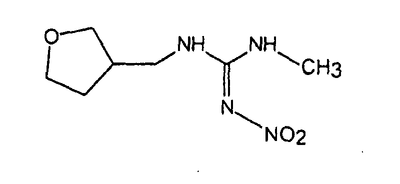 **caractérisé en ce que** l'on fait réagir un composé de la formule (IIc) : 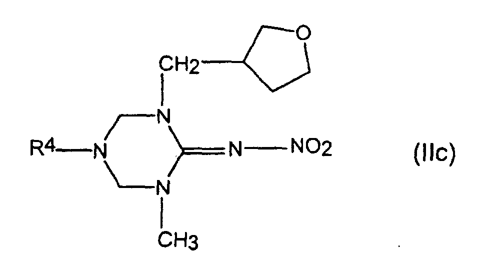 le cas échéant, en présence d'un agent de dilution avec un composé de la formule (III) :
R⁵ -NH₂
où R⁴ = R⁵ représente le radical méthyle, éthyle, cyclopropyle, cyclopentyle, benzyle ou furfuryle.

8. Procédé selon la revendication 1, **caractérisé en ce que** la réaction est réalisée en l'absence d'un agent de dilution.

9. Procédé selon la revendication 1, **caractérisé en ce que** la réaction est réalisée en présence d'un agent de dilution de la série des alcools.

10. Procédé selon la revendication 1, **caractérisé en ce qu'**il est réalisé à une température allant de 0°C à 200°C.
